# EUROPÄISCHE PATENTSCHRIFT

(11) **EP 3 541 751 B1**
(45) Veröffentlichungstag und Bekanntmachung des Hinweises auf die Patenterteilung: **25.01.2023**
(21) Anmeldenummer: 17807742.6
(22) Anmeldetag: 13.11.2017
(51) Int. Cl.: C01C 1/04, C07C 273/04, C07C 273/10, C01B 3/52

(54) **VERFAHREN ZUR HERSTELLUNG VON AMMONIAK UND HARNSTOFF IN EINEM GEMEINSAMEN ANLAGENKOMPLEX**
METHOD FOR PRODUCING AMMONIA AND UREA IN A COMMON FACILITY
PROCÉDÉ DE PRODUCTION D'AMMONIAC ET D'URÉE DANS UN COMPLEXE D'INSTALLATIONS COMMUN

(30) Priorität: 21.11.2016 DE 102016122374
(43) Veröffentlichungstag der Anmeldung: 25.09.2019
(73) Patentinhaber: thyssenkrupp Industrial Solutions AG, 45143 Essen (DE); thyssenkrupp AG, 45143 Essen (DE)
(72) Erfinder: JOHANNING, Joachim, 46045 Oberhausen (DE); MAKHYNYA, Yevgeny, 45481 Mülheim an der Ruhr (DE)
(74) Vertreter: thyssenkrupp Intellectual Property GmbH
(86) Internationale Anmeldenummer: PCT/EP2017/079053
(87) Internationale Veröffentlichungsnummer: WO 2018/091410

(56) Entgegenhaltungen:
- DE-A1- 1 668 547
- US-A- 3 310 376
- US-A- 3 640 052
- US-A- 4 320 103

## Beschreibung

Die Erfindung betrifft ein Verfahren zur Herstellung von Ammoniak und Harnstoff in einem gemeinsamen Anlagenkomplex, wobei ein Rohsynthesegasstrom, welcher mindestens die Gase Wasserstoff, Stickstoff und Kohlendioxid enthält, zunächst komprimiert wird, danach mindestens ein Teilstrom des Rohsynthesegases mit Ammoniak gewaschen wird, wobei ein an Kohlendioxid abgereicherter gereinigter Synthesegasstrom gebildet wird sowie ein Kondensat, wobei aus dem an Kohlendioxid abgereicherten gereinigten Synthesegasstrom Ammoniak synthetisiert wird und aus dem Kondensat Harnstoff synthetisiert wird unter Bildung einer wässrigen Harnstoffzusammensetzung. Die Erfindung betrifft weiterhin eine Anlage zur Synthese von Ammoniak und Harnstoff, wobei die Edukte der Harnstoffsynthese ganz aus Anlagen zur Herstellung von Ammoniak bereitgestellt werden können.

Die großtechnische Herstellung von Harnstoff beruht derzeit praktisch ausschließlich auf der Hochdrucksynthese von Ammoniak (NH₃) und Kohlendioxid (CO₂) bei ca. 150 bar und ca. 180 °C. Beide Edukte kommen in der Regel aus einer Ammoniak-Anlage, die meist in enger Nachbarschaft zu der betreffenden Harnstoff-Anlage steht.

In der Synthesegaserzeugung der Ammoniak-Anlage fällt das für die Harnstoff-Synthese benötigte Kohlendioxid als Bestandteil des Rohsynthesegases nach der Reformierung an. Da es in der Ammoniak-Synthese als Katalysatorgift wirken würde, muss das ganze Kohlendioxid - bis auf einen Grenzwert von 500 ppm - aus dem Synthesegas abgetrennt werden. Stand der Technik ist hierfür der Einsatz regenerativer Gaswäschen, für die eine größere Zahl selektiv wirkender Lösungsmittel zur Verfügung steht.

Kohlendioxid kann auch mit Hilfe eines Ammoniak/Wasser-Gemisches oder mit Hilfe von Ammoniak abgetrennt werden. Wird das Kohlendioxid mit Hilfe eines Ammoniak/WasserGemisches abgetrennt, so wird es in der Lösung vorwiegend chemisch in Form von Carbamat-, Hydrogenkarbonat- und Karbonat-Ionen gebunden.

Bei dem direkten Kontakt des Kohlendioxids mit Ammoniak entsteht überwiegend Ammoniumcarbamat, dieses ist gleichzeitig das Edukt der Harnstoffsynthese. Für Ammoniumcarbamat existiert nur eine theoretische Speicherungsmöglichkeit, da es generell zur Degradation neigt. Diese Neigung wird durch Wasseranwesenheit noch beschleunigt.

Die Harnstoffsynthese aus Ammoniak und Kohlendioxid ist insgesamt exotherm. Sie besteht aus der relativ starken exothermen und verhältnismäßig schnellen Reaktion der Edukte zu Ammoniumcarbamat sowie der deutlich langsameren und endothermen Weiterreaktion zu Harnstoff und Wasser. Eine gute Energieeffizienz des Gesamtverfahrens lässt sich nur erreichen, wenn die freiwerdende Reaktionswärme der relativ stark exothermen Carbamatbildungsreaktion für die folgende Harnstoffbildung zumindest anteilig genutzt wird. Die Speicherung des Carbamat-Zwischenproduktes dagegen impliziert die Abkühlung des Reaktionsgemisches und führt zu einem zusätzlichen Energieaufwand. Auf diesem Weg kann keine günstige Gesamtlösung realisiert werden.

In der Vergangenheit wurden etliche Konzepte für eine prozessseitige Integration von Ammoniak-Anlage einerseits und Harnstoff-Anlage andererseits vorgeschlagen, die ältesten Konzepte wurden bereits Anfang des 20. Jahrhunderts veröffentlicht. Die meisten Konzepte beruhen auf der Abtrennung des Kohlendioxids aus dem Rohsynthesegas der Ammoniak-Anlage mit prozesseigenem Ammoniak oder mit Ammoniak/Wasser-Gemischen und weiterem Transfer des überwiegend als Ammoniumcarbamat vorliegenden Produktes zum Harnstoff-Reaktor.

Bisher wurde allerdings noch keines dieser Konzepte großtechnisch realisiert. Gründe hierfür sind nicht bekannt geworden.

Offenbar ist gemäß den herkömmlichen Konzepten bei der Abtrennung des Kohlendioxids mit prozesseigenem Ammoniak der Betrieb der Ammoniak- und der Harnstoff-Anlage nur simultan möglich. Dabei wird zwangsläufig das ganze während der Dampfreformierung entstehende Kohlendioxid in die Harnstoffsynthese weitergeleitet und umgesetzt, was den Gesamtkomplex unflexibel macht und in Richtung "Balanced Plant" bewegt. In diesem Fall ist die Herstellung weiterer Produkte, wie z.B. Salpetersäure, in nennenswerten Mengen nicht möglich.

Zudem wäre ein - mit der konventionellen Prozessführung möglicher - separater Betrieb einer der beiden Anlagen kaum noch realisierbar. Außerdem kommen noch chemische Probleme, wie z.B. ein zusätzlicher Wassereintrag in die Harnstoffsynthese, hinzu, wodurch das Gleichgewicht der Harnstoff-Bildungsreaktion ungünstig beeinflusst wird.

Bisher sind zudem keine Verfahren und keine Vorrichtungen bekannt, welche eine separate Inbetriebnahme der Ammoniak-, bzw. Harnstoff-Anlage oder einen flexiblen Betrieb integrierter Anlagen ermöglichen.

DE 1 668 547 offenbart ein Verfahren zur Herstellung von NH₃ und Harnstoff, das dadurch gekennzeichnet ist, dass man CO₂, N₂ und H₂ enthaltendes NH₃-Synthesegas in eine erste Zone leitet, die unter solchen Bedingungen gehalten wird, dass CO₂ aus dem Synthesegas in einer ammoniakhaltigen Flüssigkeit abgetrennt und ein Ammoniumcarbamat enthaltendes Kondensat erhalten wird, und dass man das restliche Ammoniaksynthesegas in eine Ammoniaksynthesezone leitet und das Kondensat in eine zweite Zone leitet, die unter zur Herstellung von Harnstoff aus dem Kondensat geeigneten Bedingungen gehalten wird. Das ist ein typisches Beispiel eines vollintegrierten Verfahrens, bei dem keine direkte CO₂-Abtrennung stattfindet.

DE 25 26 353 betrifft ein integriertes Verfahren zur Herstellung von Harnstoff und NH₃. Dabei erfolgt eine Entfernung des NH₃ aus dem Kreislaufgas durch Wasserwäsche. CO₂ wird aus dem rohen Synthesegas durch NH₃/H₂O-Wäsche ausgewaschen.

DE 26 15 102 C2 offenbart ein Verfahren zur gleichzeitigen Herstellung von NH₃ und Harnstoff, bei dem ein beim Reformieren von Kohlenwasserstoffen und anschließender CO-Umwandlung erhaltenes Gasgemisch aus CO₂, N₂ und H₂ einer Absorption des Kohlendioxids mittels Ammoniaklösung, die bei einer Absorption des Ammoniaks aus der Ammoniaksynthese mittels Wasser anfällt, zugeführt und die so gebildete Ammoniumcarbamatlösung in die Harnstoffsynthese geführt wird.

US 4,138,434 betrifft ein Prozesskonzept mit Integration von NH₃- und Harnstoff-Synthese. Dabei erfolgt eine zweistufige Auswaschung von CO₂ aus dem rohen Synthesegas mit wässrigem Ammoniak unter Bildung von Ammoniumcarbamat. Restliches NH₃ wird mit rohem Synthesegas ausgetrieben. Es erfolgt ein thermisches Strippen, wobei für das Kompensieren von Druckverlusten ein Ejektor eingesetzt wird.

Aus der EP 1 188 710 A2 ist ein Verfahren für eine kombinierte Herstellung sowohl von Ammoniak als auch von Harnstoff bekannt, bei dem ein Ammoniak-Synthesegas mit einem hohen Gehalt an Kohlendioxid zunächst auf einen für die Ammoniaksynthese geeigneten Druck gebracht wird. Der überwiegende Anteil an Kohlendioxid wird durch Waschen mit einer wässrigen Ammoniaklösung entfernt, wobei eine Ammoniumcarbamat-Lösung erhalten wird, die einem Harnstoffreaktor zugeleitet wird. Aus dem an Kohlendioxid abgereicherten gestrippten Gas wird zunächst in einer Methanisierung Kohlenmonoxid entfernt. Das nach der Methanisierung mit Ammoniak gesättigte Gas wird mit einem Produktgas aus einem Ammoniakkonverter kombiniert, und dann mit Wasser gewaschen, um Ammoniak zu entfernen. Das aus der Waschvorrichtung austretende Gas enthält etwas Ammoniak und etwas Wasser, die in einem Absorber mit Molekularsieb entfernt werden. Stromabwärts des Absorbers wird das gereinigte Gas zunächst einem Kompressor und dann einer Ammoniaksyntheseeinheit zugeleitet. Das Produktgas aus der Ammoniaksynthese wird mit Wasser gewaschen. Das dabei erhaltene Ammoniak/Wasser-Gemisch wird konzentriert und nach Erhöhen der Ammoniakkonzentration zu der Stufe zurückgeführt, in der die Ammoniumcarbamat-Lösung erzeugt wird. Bei diesem bekannten Verfahren wird der gesamte Rohsynthesegasstrom einer Vorrichtung zugeführt, in der durch Umsetzung mit einem Ammoniak/Wasser-Gemisch aus dem im Rohsynthesegas enthaltenen Kohlendioxid Carbamat erzeugt wird. Erst nach dieser Abtrennung des Kohlendioxids wird dann der Gasstrom einer Methanisierung und danach einer Wäsche mit Wasser zugeführt, um Ammoniak aus dem Synthesegasstrom zu entfernen. Es folgen dann weitere Trennschritte, bevor aus dem gereinigten Synthesegas Ammoniak synthetisiert wird.

Die bekannten vollintegrierten Verfahren und Vorrichtungen sind somit nicht in jeder Hinsicht zufriedenstellend und es besteht ein Bedarf an verbesserten Verfahren und Vorrichtungen. Insbesondere sollten der Energiebedarf der Kohlendioxid-Verdichtung verringert und/oder der apparative Aufwand für die Anlage insgesamt reduziert werden.

Es ist die Aufgabe der Erfindung, ein verbessertes Verfahren und eine verbesserte Anlage zur Herstellung von Ammoniak und Harnstoff bereitzustellen, welche zur Synthese von Harnstoff und Ammoniak in einem gemeinsamen Anlagenkomplex eingesetzt werden können und die Nachteile des Standes der Technik überwinden.

Diese Aufgabe wird durch den Gegenstand der Patentansprüche 1 bezüglich des Verfahrens sowie 7 bezüglich der Anlage gelöst.

Erfindungsgemäß wird bei Herstellung von Ammoniak und Harnstoff in dem gemeinsamen Anlagenkomplex der Rohsynthesegasstrom nach der Komprimierung in zwei Teilströme aufgeteilt, nämlich einen ersten Synthesegasteilstrom und einen zweiten Synthesegasteilstrom, wobei nur der erste Synthesegasteilstrom mit flüssigem Ammoniak gewaschen wird. Diese Maßnahme hat den Vorteil, dass man den zweiten Synthesegasteilstrom, welcher auch Kohlendioxid enthält, beispielsweise zum Strippen einer wässrigen Harnstofflösung nutzen kann, welche bei der Harnstoffsynthese anfällt. Es ist somit nicht notwendig, für ein solches Strippen der Anlage zusätzliches Kohlendioxid zuzuführen. Die Herstellung von Ammoniak und Harnstoff in einem gemeinsamen Anlagenkomplex wird in der Fachwelt auch als "integrierte Synthese von Harnstoff und Ammoniak" bezeichnet.

Wenn in der vorliegenden Anmeldung von einer Aufteilung in zwei Teilströme die Rede ist, dann ist damit gemeint, dass der Synthesegasstrom bei gleichbleibender Zusammensetzung in zwei Teilströme aufgeteilt wird, die dann nach der Aufteilung über separate Leitungswege weitergeleitet und im nachfolgenden Prozess auf unterschiedliche Weise weiter behandelt werden. Es handelt sich somit bei dieser Aufteilung nicht um eine Auftrennung des Synthesegasstroms in der Weise, dass einzelne Gaskomponenten aus dem Synthesegasstrom abgetrennt und dadurch zwei Synthesegasströme unterschiedlicher Zusammensetzung erzeugt werden.

Die prozentuale Aufteilung des Synthesegasstroms in die beiden Teilströme kann selbstverständlich in weiten Bereichen variieren, das heißt es ist keineswegs so, dass eine Aufteilung in zwei Teilströme mit jeweils gleich großen Volumenanteilen erfolgt.

Die erfindungsgemäß vorgeschlagene Lösung vermeidet die Nachteile der oben beschriebenen bekannten Verfahren und ermöglicht auch eine separate Inbetriebnahme und einen separaten Betrieb der beiden Teilanlagen des Anlagenkomplexes sowie einen jeweiligen Betrieb in Teillast. Dies wird mit weiteren Anlagenteilen erreicht, die im weiteren Verlauf beschrieben werden. Durch die erfindungsgemäße Lösung werden zudem eine Reduzierung der Kapitalkosten und eine Verbesserung des spezifischen Energieverbrauchs erzielt.

Es wurde überraschend gefunden, dass das erfindungsgemäße Verfahren einen faktischen Wegfall der in der konventionellen Harnstoffsynthese erforderlichen Vorrichtung zur Kohlendioxid-Kompression ermöglicht. Außerdem fällt bei Durchführung des erfindungsgemäßen Verfahrens die bei der konventionellen Ammoniaksynthese erforderliche Vorrichtung zur Kohlendioxid-Wäsche weg.

Da der Dampfbedarf für den Kohlendioxid-Kompressor entfällt, kann sich eine signifikante Reduzierung des Energiebedarfs für den gesamten Anlagen-Komplex ergeben. Darüber hinaus führt der Wegfall des Kohlendioxid-Kompressors einschließlich seiner Zusatzeinrichtungen (Antriebsturbine, Zwischenkühler, Ölsystem, etc.) und der Kohlendioxid-Wäsche zu einer sehr substanziellen Reduzierung der Anlageninvestitionskosten.

Ferner wird durch das erfindungsgemäße Verfahren ein zusätzlicher Wassereintrag in die Harnstoff-Synthese weitgehend vermieden. Denn das Kohlendioxid kann aus der Waschlösung, welche beim Waschen mit Wasser und Ammoniak entsteht, bei einer erhöhten Temperatur und einem Druckniveau leicht oberhalb des Harnstoffsynthesedrucks ausgetrieben werden. Mit steigendem Desorptionsdruck ist es möglich das Kohlendioxid mit ausreichender Reinheit für die Harnstoffsynthese zu bekommen und insbesondere der Wasseranteil wird deutlich reduziert.

Die für die Desorption des Kohlendioxids benötigte Wärmemenge sowie die für den Mehrbedarf des Synthesegaskompressors benötigte Dampfmenge, sowie weitere Dampf- und Wärmemengen, können mit begrenzten Modifikationen des Dampfsystems in Ammoniak/Harnstoff-Komplexen verfügbar gemacht werden. Des Weiteren kann auch die von der Kohlendioxid-Wäsche in der konventionellen Prozessführung beanspruchte Wärme durch das Dampfsystem genutzt werden.

Eine Weiterbildung der Erfindung betrifft ein Verfahren zur integrierten Herstellung von Ammoniak und Harnstoff, wobei das erfindungsgemäße Verfahren die folgenden Schritte umfasst:
a. Bereitstellen eines Rohsynthesegasstroms umfassend Wasserstoff, Stickstoff und Kohlendioxid sowie gegebenenfalls geringere Anteile an Methan, Argon, Kohlenmonoxid und evtl. Helium;
b. gegebenenfalls Aufteilen des Rohsynthesegasstroms in einen ersten Rohsynthesegasstrom und in einen dritten Rohsynthesegasstrom;
c. gegebenenfalls Waschen des dritten Rohsynthesegasstroms mit einer Waschlösung umfassend Wasser und Ammoniak unter Bildung eines an Kohlendioxid abgereicherten vorgereinigten Synthesegasstroms und einer an Kohlendioxid angereicherten Waschlösung;
d. gegebenenfalls Austreiben von Kohlendioxid aus der an Kohlendioxid angereicherten Waschlösung unter Bildung eines komprimierten Kohlendioxidstroms;
e. gegebenenfalls Vereinen des vorgereinigten Synthesegasstroms mit dem dritten Rohsynthesegasstrom unter Bildung eines vereinten Synthesegasstroms;
f. Komprimieren des gegebenenfalls vereinten Synthesegasstroms unter Bildung eines komprimierten Synthesegasstroms;
g. Aufteilen des komprimierten Synthesegasstroms in einen ersten Synthesegasstrom und in einen zweiten Synthesegasstrom;
h. Bereitstellen einer Zusammensetzung umfassend flüssigen Ammoniak;
i. Waschen des ersten Synthesegasstroms und gegebenenfalls des Strippgasstroms aus Schritt (k), sowie gegebenenfalls des komprimierten Kohlendioxidstroms aus Schritt (d) mit der Zusammensetzung umfassend flüssigen Ammoniak unter Bildung eines an Kohlendioxid abgereicherten gereinigten Synthesegasstroms und eines Kondensats;
j. Synthetisieren von
   i. (j₁) Ammoniak aus dem an Kohlendioxid abgereicherten gereinigten Synthesegasstrom; und
   ii. (j₂) Harnstoff aus dem Kondensat unter Bildung einer wässrigen Harnstoffzusammensetzung;
   iii. (k) gegebenenfalls Strippen der wässrigen Harnstoffzusammensetzung aus Schritt (j2) mit Hilfe des zweiten Synthesegasstroms aus Schritt (g).

Bei dieser möglichen Variante der Erfindung wird der Rohsynthesegasstrom bereits vor der Komprimierung in zwei Teilströme aufgeteilt, unter Bildung eines dritten (Roh)Synthesegasteilstroms und eines Rohsynthesegasstroms, welcher einem Kompressor zugeführt wird, wobei der dritte (Roh)Synthesegasteilstrom in einer Waschvorrichtung mit einer Waschlösung umfassend Wasser und Ammoniak gewaschen wird unter Bildung eines an Kohlendioxid abgereicherten vorgereinigten Synthesegasstroms und einer an Kohlendioxid angereicherten Waschlösung, wobei der vorgereinigte Synthesegasstrom danach wieder mit dem Rohsynthesegasstrom vereinigt und mit diesem gemeinsam dem Kompressor zugeführt wird.

Aus der Waschlösung kann in einer Desorptionsvorrichtung Kohlendioxid ausgetrieben werden auf einen Druck, der oberhalb des Niveaus der Harnstoffsynthese liegt.

Das erfindungsgemäße Verfahren dient der Herstellung von Ammoniak und Harnstoff. Dabei erfolgt vorzugsweise die integrierte Herstellung von Ammoniak und Harnstoff, d.h. zumindest ein Teil des hergestellten Ammoniaks und/oder zumindest ein Teil des bei der Herstellung des Ammoniaks angefallenen Kohlendioxids werden zur anschließenden Herstellung von Harnstoff eingesetzt. Es ist jedoch erfindungsgemäß auch möglich, zumindest zeitweise ausschließlich Ammoniak herzustellen.

Das erfindungsgemäße Verfahren ermöglicht die Synthese von Ammoniak und Harnstoff in einem vollintegrierten Prozess. Zum Zwecke der Beschreibung betrifft nachfolgend der Begriff "Ammoniak-Anlage" alle Vorrichtungen, welche gesondert zur Synthese von Ammoniak eingesetzt werden, und der Begriff "Harnstoff-Anlage" alle Vorrichtungen, welche gesondert zur Synthese von Harnstoff eingesetzt werden.

Das Konzept ermöglicht vier verschiedene Betriebsweisen:
i. den Betrieb beider Anlagen im vollintegrierten Modus bei Voll- oder Teillast (Fall I);
ii. den alleinigen Betrieb der Ammoniak-Anlage (Fall II) (nicht zur Erfindung gehörend);
iii. den Betrieb beider Anlagen in einem alternativen vollintegrierten Modus (Fall III);
iv. den alleinigen Betrieb der Ammoniak-Anlage in einem alternativen vollintegrierten Modus (Fall IV) (nicht zur Erfindung gehörend).

D.h. die Ammoniak-Anlage wird in allen vier Fällen im Voll- oder Teillast-Betrieb betrieben, während gleichzeitig die Harnstoff-Anlage entweder in Voll- oder Teillast (Fälle I und III) oder gar nicht betrieben wird (Fälle II und IV) (nicht zur Erfindung gehörend).

In Schritt (a) des erfindungsgemäßen Verfahrens wird ein Rohsynthesegasstrom bereitgestellt, welcher Wasserstoff, Stickstoff und Kohlendioxid sowie gegebenenfalls geringere Anteile an Methan, Argon, Kohlenmonoxid und evtl. Helium umfasst.

Bei dem erfindungsgemäßen Verfahren wird in Schritt (a) ein Synthesegas bereitgestellt, welches erfindungsgemäß als *"Rohsynthesegasstrom"* bezeichnet wird. Dieser Rohsynthesegasstrom unterscheidet sich hinsichtlich seiner Zusammensetzung, insbesondere hinsichtlich seines Gehalts an Kohlendioxid, von dem gemäß Schritt (c) erhaltenen *"vorgereinigten Synthesegasstrom"* und von dem gemäß Schritt (i) erhaltenen *"gereinigten Synthesegasstrom".* Grundsätzlich nimmt im Verlauf des erfindungsgemäßen Verfahrens der Gehalt an Kohlendioxid im Synthesegas kontinuierlich immer weiter ab.

Dabei sind H₂, N₂ und CO₂ bevorzugt die Hauptbestandteile des Rohsynthesegasstroms. Gegebenenfalls kann der Rohsynthesegasstrom weitere, bevorzugt inerte Komponenten wie Methan, Argon, Kohlenmonoxid und/oder Helium umfassen. Der Rohsynthesegasstrom wird bevorzugt aus Kohlenwasserstoffen, vorzugsweise aus Erdgas und Wasser in Form von Dampf gewonnen. Geeignete Verfahren zur Erzeugung eines solchen Rohsynthesegasstroms sind einem Fachmann bekannt und es kann diesbezüglich beispielsweise vollumfänglich verwiesen werden auf A. Nielsen, I. Dybkjaer, Ammonia - Catalysis and Manufacture, Springer Berlin 1995, Kapitel 6, Seiten 202-326; M. Appl, Ammonia. Principles and Industrial Practice, WILEY-VCH Verlag GmbH 1999. In einer bevorzugten Ausführungsform wird zumindest ein Teil des Rohsynthesegasstroms durch Dampfreformierung und/oder durch autotherme Reformierung bereitgestellt.

Der in Schritt (a) des erfindungsgemäßen Verfahrens bereitgestellte Synthesegasstrom kann bereits herkömmlichen Aufbereitungsmaßnahmen für Synthesegas unterzogen worden sein, wie z.B. Heliumentfernung, Erdgasentschwefelung und/oder Konvertierung von CO zu CO₂.

Bevorzugt wurde der in Schritt (a) bereitgestellte Rohsynthesegasstrom zuvor bereits zumindest einer solchen Konvertierung von CO zu CO₂ unterzogen.

Besonders bevorzugt wurde der Rohsynthesegasstrom jedoch zuvor keinen weiteren Aufbereitungsmaßnahmen unterzogen, welche bei der herkömmlichen Bereitstellung von Synthesegas üblicherweise auf eine solche Konvertierung von CO zu CO₂ folgen, wie z.B. CO₂-Entfernung oder Endreinigung. Für eine Endreinigung kommen in herkömmlichen Prozessen insbesondere Methanisierung, Methanolisierung, kryogene Reinigung (Stickstoffwäsche), Kupferwäsche oder deren Kombinationen in Betracht. Solchen weiteren Aufbereitungsmaßnahmen wurde der in Schritt (a) bereitgestellte Rohsynthesegasstrom bevorzugt jedoch nicht unterzogen.

Der Gehalt an CO₂ im Rohsynthesegasstrom liegt bevorzugt im Bereich von 1 Vol.-% bis 25 Vol.-%, bevorzugter von 10 Vol.-% bis 20 Vol.-%.

Bevorzugt wird der Rohsynthesegasstrom einer Wärmeintegration unterzogen. Besonders bevorzugt erfolgt die Wärmeintegration nach der Konvertierung von CO zu CO₂.

In Schritt (b) des erfindungsgemäßen Verfahrens wird der Rohsynthesegasstrom in einer Ausgestaltung des Verfahrens in einen ersten Rohsynthesegasstrom und in einen zweiten Rohsynthesegasstrom aufgeteilt.

In einer bevorzugten Ausführungsform ist dabei der erste Rohsynthesegasstrom größer als der zweite Rohsynthesegasstrom. In einer anderen bevorzugten Ausführungsform ist der zweite Rohsynthesegasstrom größer als der erste Rohsynthesegasstrom.

In einer bevorzugten Ausführungsform liegt das relative Verhältnis des ersten Rohsynthesegasstroms zum zweiten Rohsynthesegasstrom im Bereich von 1:20 bis 1:1, bevorzugter 1:15 bis 1:1, noch bevorzugter 1:10 bis 1:1. In einer anderen bevorzugten Ausführungsform liegt das relative Verhältnis des zweiten Rohsynthesegasstroms zum ersten Rohsynthesegasstrom im Bereich von 1:20 bis 1:1, bevorzugter 1:15 bis 1:1, noch bevorzugter 1:10 bis 1:1. In einer weiteren bevorzugten Ausführungsform liegt das relative Verhältnis des ersten Rohsynthesegasstroms zum zweiten Rohsynthesegasstrom im Bereich von 1:20 bis 20:1, bevorzugter 1:15 bis 15:1, noch bevorzugter 1:10 bis 10:1. Das Verhältnis kann im zeitlichen Verlauf des Verfahrens verändert werden.

In Schritt (c) des erfindungsgemäßen Verfahrens wird der erste Rohsynthesegasstrom in einer Ausgestaltung des Verfahrens mit einer Waschlösung, welche Wasser und Ammoniak umfasst, gewaschen. Dabei werden ein an Kohlendioxid abgereicherter vorgereinigter Synthesegasstrom und eine an Kohlendioxid angereicherte Waschlösung gebildet.

Geeignete Waschkolonnen zum Waschen von Synthesegas mit einer Waschlösung, welche Wasser und Ammoniak umfasst, sind einem Fachmann bekannt.

In der Waschlösung für Schritt (c), welche Wasser und Ammoniak umfasst, liegt der Anteil an Ammoniak bevorzugt im Bereich von 1 bis 50 Gew.-%, bevorzugter im Bereich von 5 bis 45 Gew.-%, noch bevorzugter im Bereich von 10 bis 40 Gew.-%, am bevorzugtesten im Bereich von 15 bis 35 Gew.-%, und insbesondere im Bereich von 20 bis 25 Gew.-%.

Die an Kohlendioxid angereicherte Waschlösung liegt bevorzugt flüssig vor und kann von dem an Kohlendioxid abgereicherten vorgereinigten Synthesegasstrom abgetrennt werden. Bevorzugt ist das Kohlendioxid in der Waschlösung vorwiegend chemisch in Form von Carbamat-, Hydrogencarbonat- und Carbonat gebunden.

Der an Kohlendioxid abgereicherte vorgereinigte Synthesegasstrom enthält bevorzugt höchstens 5 Vol.-%, bevorzugter höchstens 10 Vol.-%, noch bevorzugter höchstens 15 Vol.-%, besonders bevorzugt höchstens 20 Vol.-% Kohlendioxid.

Erfindungsgemäß ist der Anteil an Kohlendioxid im vorgereinigten Synthesegasstrom geringer als im Rohsynthesegasstrom.

In Schritt (d) des erfindungsgemäßen Verfahrens wird in einer Ausgestaltung des Verfahrens aus der an Kohlendioxid angereicherten Waschlösung Kohlendioxid ausgetrieben, wobei ein komprimierter Kohlendioxidstrom gebildet wird.

Bevorzugt wird das Kohlendioxid aus der Waschlösung aus Schritt (c) bei einer erhöhten Temperatur und einem Druckniveau leicht oberhalb des Harnstoff-Synthesedrucks ausgetrieben. Untersuchungen haben ergeben, dass es möglich ist, das Kohlendioxid auf diesem hohen Druckniveau mit ausreichender Reinheit für die Harnstoffsynthese zu bekommen und das dabei insbesondere der Wasseranteil deutlich reduziert werden kann. Wird der Kohlendioxidstrom in einem weiteren Schritt der Harnstoffsynthese zugeführt, wird ein zusätzlicher Wassereintrag in die Harnstoff-Synthese weitgehend vermieden.

In einer bevorzugten Ausführungsform kann die für die Desorption des Kohlendioxids benötigte Wärmemenge mit begrenzten Modifikationen des Dampfsystems in Ammoniak/Harnstoff-Komplexen verfügbar gemacht werden. Noch bevorzugter kann auch die Wärme, die bei einer konventionellen Prozessführung von der Kohlendioxid-Wäsche beansprucht würde, durch das Dampfsystem genutzt werden.

Bevorzugt wird der komprimierte Kohlendioxidstrom in einer Vorrichtung, der Desorptionskolonne, gebildet, die von der Vorrichtung, in der Schritt (c) des erfindungsgemäßen Verfahrens stattfindet, der Absorptionskolonne, abweicht. Die Waschkolonne für Schritt (c) und die Desorptionskolonne für Schritt (d) sind in einer gemeinsamen Vorrichtung, der AmmoniakWasser-Wäsche, vereint.

Der komprimierte Kohlendioxidstrom enthält bevorzugt mindestens 10 Vol.-%, bevorzugter mindestens 15 Vol.-%, noch bevorzugter mindestens 20 Vol.-%, besonders bevorzugt mindestens 25 Vol.-%. Kohlendioxid. Darüber hinaus kann der komprimierte Kohlendioxidstrom Ammoniak und Wasser umfassen.

In Schritt (e) des erfindungsgemäßen Verfahrens wird der vorgereinigte Synthesegasstrom aus Schritt (c) in einer Ausgestaltung des Verfahrens mit dem zweiten Rohsynthesegasstrom aus Schritt (b) unter Bildung eines vereinten Synthesegasstroms vereint.

In einer bevorzugten Ausführungsform ist dabei der vorgereinigte Synthesegasstrom größer als der zweite Rohsynthesegasstrom. In einer bevorzugten Ausführungsform ist dabei der vorgereinigte Synthesegasstrom kleiner als der zweite Rohsynthesegasstrom.

In einer bevorzugten Ausführungsform liegt das relative Verhältnis des vorgereinigten Synthesegasstroms und des zweiten Rohsynthesegasstroms im Bereich von 1:20 bis 1:1, bevorzugter 1:15 bis 1:1, noch bevorzugter 1:10 bis 1:1. In einer anderen bevorzugten Ausführungsform liegt das relative Verhältnis des vorgereinigten Synthesegasstroms und des zweiten Rohsynthesegasstroms im Bereich von 20:1 bis 1:1, bevorzugter 15:1 bis 1:1, noch bevorzugter 10:1 bis 1:1. In einer weiteren bevorzugten Ausführungsform liegt das relative Verhältnis des vorgereinigten Synthesegasstroms und des zweiten Rohsynthesegasstroms im Bereich von 1:20 bis 20:1, bevorzugter 1:15 bis 15:1, noch bevorzugter 1:10 bis 10:1. Das Verhältnis kann im zeitlichen Verlauf des Verfahrens verändert werden.

Bevorzugt ist der Anteil an Kohlendioxid im vereinten Synthesegasstrom geringer als im Rohsynthesegasstrom, aber höher als im vorgereinigten ersten Synthesegasstrom.

Der Gehalt an Kohlendioxid im vereinten Synthesegasstrom beträgt bevorzugt mindestens 5 Vol.-%, bevorzugter mindestens 10 Vol.-%, noch bevorzugter mindestens 15 Vol.-%, besonders bevorzugt mindestens 20 Vol.-%. Kohlendioxid. In einer weiteren Ausführungsform kann der vereinte Synthesegasstrom weitere, bevorzugt inerte Komponenten wie Methan, Argon, Kohlenmonoxid und/oder Helium umfassen.

In Schritt (f) des erfindungsgemäßen Verfahrens wird der vereinte Synthesegasstrom aus Schritt (e) unter Bildung eines komprimierten Synthesegasstroms komprimiert.

Nach Schritt (f) des erfindungsgemäßen Verfahrens erfolgen bevorzugt alle weiteren Schritte (g) bis (i) und die Harnstoffsynthese in Schritt (j₂) auf einem gemeinsamen Druckniveau von jeweils mindestens 145 bar oder bei einem Druck, welcher leicht oberhalb des Synthesedrucks des Harnstoffs liegt. Dabei sind bei der Durchführung der weiteren Schritte (g) bis (i) und (j₂) Druckverluste möglich, die üblicherweise bei der großtechnischen Harnstoffsynthese anfallen. Das Druckniveau, das in Schritt (f) des erfindungsgemäßen Verfahrens erreicht wird, ist bevorzugt ausreichend hoch um diese Druckverluste zu überwinden.

Die Ammoniaksynthese in Schritt (j₁) kann jedoch vorzugsweise auch bei einem deutlich höheren Druckniveau, z.B. von 200 bis 350 bar, durchgeführt werden.

In einer weiteren bevorzugten Ausführungsform werden nach Schritt (f) des erfindungsgemäßen Verfahrens alle weiteren Schritte (g) bis (j) auf einem gemeinsamen Druckniveau durchgeführt. Bevorzugter wird dabei im Anschluss an Schritt (f) bis Schritt (j) des erfindungsgemäßen Verfahrens keine weitere Verdichtung der Gasströme durchgeführt.

Besonders bevorzugt ist eine Ausführungsform, bei der der Gasstrom im Anschluss an Schritt (d) vor Durchführung der Harnstoff-Synthese nicht erneut durch einen CO₂-Kompressor verdichtet wird.

In Schritt (g) des erfindungsgemäßen Verfahrens wird der komprimierte Synthesegasstrom aus Schritt (f) in einen ersten Synthesegasstrom und in einen zweiten Synthesegasstrom aufgeteilt.

In einer bevorzugten Ausführungsform ist dabei der erste Synthesegasstrom größer als der zweite Synthesegasstrom. In einer bevorzugten Ausführungsform ist dabei der erste Synthesegasstrom kleiner als der zweite Synthesegasstrom.

In einer bevorzugten Ausführungsform liegt das relative Verhältnis des ersten Synthesegasstroms und des zweiten Synthesegasstroms im Bereich von 1:20 bis 1:1, bevorzugter 1:15 bis 1:1, noch bevorzugter 1:10 bis 1:1. In einer anderen bevorzugten Ausführungsform liegt das relative Verhältnis des ersten Synthesegasstroms und des zweiten Synthesegasstroms im Bereich von 20:1 bis 1:1, bevorzugter 15:1 bis 1:1, noch bevorzugter 10:1 bis 1:1. In einer weiteren bevorzugten Ausführungsform liegt das relative Verhältnis des ersten Synthesegasstroms und des zweiten Synthesegasstroms im Bereich von 1:20 bis 20:1, bevorzugter 1:15 bis 15:1, noch bevorzugter 1:10 bis 10:1. Das Verhältnis kann im zeitlichen Verlauf des Verfahrens verändert werden.

In Schritt (h) des erfindungsgemäßen Verfahrens wird eine Zusammensetzung bereitgestellt, welche flüssigen Ammoniak umfasst.

Bevorzugt wird der flüssige Ammoniak für Schritt (h) vollständig durch Schritt (j₁) bereitgestellt.

In einer anderen bevorzugten Ausführungsform wird in Schritt (k) die wässrige Harnstoffzusammensetzung aus Schritt (j₂) durch Strippen mit Hilfe des zweiten Synthesegasstroms aus Schritt (g) weiter aufgereinigt. Der zweite Synthesegasstrom wird anschließend bevorzugt mit dem ersten Synthesegasstrom und ggf. dem komprimierten Kohlendioxidstrom aus Schritt (d) in Schritt (i) vereinigt.

In Schritt (i) des erfindungsgemäßen Verfahrens werden der erste Synthesegasstrom aus Schritt (g), gegebenenfalls der Strippgasstrom aus Schritt (k), sowie gegebenenfalls der komprimierte Kohlendioxidstrom aus Schritt (d) mit der Zusammensetzung, welche flüssigen Ammoniak umfasst, aus Schritt (h) gewaschen. Dabei werden ein an Kohlendioxid abgereicherter gereinigter Synthesegasstrom und ein Kondensat gebildet.

Geeignete Waschkolonnen zum Waschen von Synthesegas mit einer Zusammensetzung, welche flüssigen Ammoniak umfasst, sind einem Fachmann bekannt.

Das Kondensat liegt flüssig vor und kann von dem gereinigten Synthesegasstrom getrennt werden. In einer bevorzugten Ausführungsform wird das Kondensat in der Waschkolonne, in der Schritt (i) durchgeführt wird, von dem gereinigten Synthesegasstrom getrennt.

In dem Kondensat liegt das Kohlendioxid bevorzugt vorwiegend in Form von Ammoniumcarbamat vor, welches das Edukt für die Harnstoffsynthese ist. Darüber hinaus kann das Kondensat andere Ammoniumsalze wie Ammoniumhydrogencarbonat und Ammoniumcarbonat sowie nicht reagiertes NH₃ und CO₂ umfassen.

Der an Kohlendioxid abgereicherte gereinigte Synthesegasstrom kann noch Reste von CO₂ und/oder CO enthalten. Bevorzugt enthält der gereinigte Synthesegasstrom höchstens 0,05 mol.-% Kohlendioxid, noch bevorzugter höchstens 0,04 mol.-%, besonders bevorzugt höchstens 0,05 mol.-%. Erfindungsgemäß ist der Anteil an Kohlendioxid im gereinigten Synthesegasstrom geringer als im ersten Synthesegasstrom. Der Anteil an Kohlendioxid im gereinigten Synthesegasstrom liegt unter 500 ppm.

In einer bevorzugten Ausführungsform wird der in der konventionellen Harnstoff-Synthese benötigte Carbamatkondenser durch das Waschen mit der Zusammensetzung, welche flüssigen Ammoniak umfasst, gemäß Schritt (i) des erfindungsgemäßen Verfahrens, ersetzt.

In Schritt (j) des erfindungsgemäßen Verfahrens werden Ammoniak und Harnstoff synthetisiert. Dabei wird in Schritt (j₁) Ammoniak aus dem an Kohlendioxid abgereicherten gereinigten Synthesegasstrom aus Schritt (i) synthetisiert. Harnstoff wird dabei in Schritt (j₂) aus dem Kondensat aus Schritt (i) synthetisiert, wobei eine wässrige Harnstoffzusammensetzung gebildet wird.

Die Synthese von Harnstoff in Schritt (j₂) erfolgt bevorzugt in einem herkömmlichen Harnstoffreaktor, dessen Aufbau einem Fachmann bekannt ist.

In einer bevorzugten Ausführungsform umfasst das Synthetisieren des Harnstoffs in Schritt (j₂) nur die endotherme Teilreaktion bei der das Ammoniumcarbamat zu Harnstoff und Wasser umgesetzt wird.

Da das Ammoniumcarbamat in Schritt (i) bereits gebildet wurde, kann die exotherme Reaktion zur Bildung von Ammoniumcarbamat nicht mehr den Energiebedarf für die endotherme Reaktion in vollem Umfang abdecken, so dass der Harnstoffreaktor bevorzugt zusätzlich von außen beheizt wird.

Bevorzugt erfolgt die Synthese von Harnstoff in Schritt (j₂) bei einem Druck im Bereich von 100 bis 300 bar, bevorzugter im Bereich von 120 bis 200 bar, noch bevorzugter im Bereich von 140 bis 160 bar.

Die Synthese von Ammoniak aus dem gereinigten Synthesegasstrom in Schritt (j₁) erfolgt bevorzugt in einem herkömmlichen Ammoniakreaktor, dessen Aufbau einem Fachmann bekannt ist.

In einer bevorzugten Ausführungsform wird der gereinigte Synthesegasstrom aus Schritt (i) vor der Ammoniaksynthese einem weiteren Reinigungsschritt, z.B. Methanisierung, unterzogen. Diese Verfahren sind Stand der Technik der Ammoniaksynthese und dem Fachmann bekannt.

Bevorzugt wird zumindest ein Teil des im Ammoniakreaktor gebildeten gasförmigen NH₃ auskondensiert, wozu der Gasstrom bevorzugt zunächst einen Wärmetauscher und anschließend eine Kondensationsvorrichtung durchläuft. Dabei wird der Gasstrom abgekühlt, bevorzugt auf Temperaturen im Bereich von +5 °C bis -40 °C, bevorzugter im Bereich von 0 °C bis -35 °C, im Bereich von -5 °C bis -33 °C oder im Bereich von -10 °C bis -30 °C.

Bei einem nicht zur Erfindung gehörenden Verfahren entfällt eine konventionelle CO₂-Wäsche der Ammoniak-Anlage; diese wird durch das Waschen des Rohsynthesegasstroms mit Waschlösungen umfassend Wasser und Ammoniak gemäß Schritt (c) und/oder flüssigem Ammoniak gemäß Schritt (i) ersetzt. Ebenso entfällt ein ansonsten für die Harnstoffsynthese erforderlicher CO₂-Verdichter.

In einer bevorzugten Ausführungsform umfasst das erfindungsgemäße Verfahren den zusätzlichen Schritt (k), bei dem die wässrige Harnstoffzusammensetzung aus Schritt (j₂) mit Hilfe des zweiten Synthesegasstroms aus Schritt (g) gestrippt wird. Dabei werden ein Strippgasstrom und eine gereinigte wässrige Harnstofflösung gebildet.

Die in Schritt (j₂) hergestellte wässrige Harnstoffzusammensetzung umfasst im Wesentlichen Harnstoff, Wasser, Ammoniumcarbamat, andere Ammoniumsalze wie Ammoniumhydrogencarbonat und Ammoniumcarbonat sowie nicht reagiertes NH₃ und CO₂. Das Ammoniumcarbamat soll thermisch zersetzt werden und das NH₃ sowie das CO₂ müssen aus der Lösung entfernt werden und können danach erneut für das Reaktionsverfahren zur Harnstoffsynthese benutzt werden. Das Entfernen wird bevorzugt durch Strippen mit dem zweiten Synthesegasstrom aus Schritt (g) gemäß Schritt (k) erreicht. Strippingverfahren sind Stand der Technik bei Harnstoffanlagen und dem Fachmann bekannt.

Vorzugsweise wird bei dem Waschen in Schritt (i) des erfindungsgemäßen Verfahrens eine Zusammensetzung eingesetzt, welche sich ergibt durch das Vereinen
- der zuvor in Schritt (h) bereitgestellten Zusammensetzung umfassend flüssigen Ammoniak, sowie
- des Strippgasstroms aus Schritt (k).

In einer weiteren bevorzugten Ausführungsform des erfindungsgemäßen Verfahrens wird der Strippgasstrom aus Schritt (k) mit dem ersten Synthesegasstrom und gegebenenfalls zumindest einem Teil des komprimierten Kohlendioxidstroms aus Schritt (d) in Schritt (i) zusammengeführt und mit der Zusammensetzung umfassend flüssigen Ammoniak aus Schritt (h) vereint.

Bevorzugt werden die Bestandteile des Strippgasstroms durch die Vereinigung mit der Zusammensetzung umfassend flüssigen Ammoniak wieder der Harnstoffsynthese zugeführt.

In einer besonders bevorzugten Ausführungsform werden beim Waschen gemäß Schritt (i) die folgenden Komponenten zu einem gereinigten Synthesegasstrom und einem Kondensat umgesetzt:
- die zuvor in Schritt (h) bereitgestellte Zusammensetzung umfassend flüssigen Ammoniak,
- der erste Synthesegasstrom aus Schritt (g),
- bevorzugt der Strippgasstrom aus Schritt (k), und
- gegebenenfalls zumindest einem Teil des komprimierten Kohlendioxidstroms aus Schritt (d).

In einer bevorzugten Ausführungsform umfasst das erfindungsgemäße Verfahren den zusätzlichen Schritt (i*), bei dem der an Kohlendioxid abgereicherte gereinigte Synthesegasstrom aus Schritt (i) unter Bildung eines Reinstsynthesegasstroms methanisiert wird und der Reinstsynthesegasstrom zum Synthetisieren von Ammoniak gemäß Schritt (j₁) überführt wird. Geeignete Verfahren zur Hydrierung von CO und CO₂ zu Methan sind einem Fachmann bekannt.

Der Gehalt an CO₂ im Reinstsynthesegasstrom aus Schritt (i*) ist erfindungsgemäß geringer als im gereinigten Synthesegasstrom und liegt bevorzugt im Bereich bis 500 ppm bevorzugter unterhalb von 500 ppm. Noch bevorzugter umfasst der Reinstsynthesegasstrom im Wesentlichen nur noch H₂ und N₂ sowie Inertgase.

In einer bevorzugten Ausführungsform wird der Reinstsynthesegasstrom der Synthese von NH₃ gemäß Schritt (j₁) zugeführt.

In einer bevorzugten Ausführungsform umfasst das erfindungsgemäße Verfahren den zusätzlichen Schritt (d*), bei dem zumindest ein Teil des komprimierten Kohlendioxidstroms aus Schritt (d) mit der Zusammensetzung umfassend flüssigen Ammoniak aus Schritt (h) vereint wird.

In einer bevorzugten Ausführungsform des erfindungsgemäßen Verfahrens wird die Gesamtmenge des komprimierten Kohlendioxidstroms aus Schritt (d) mit der Zusammensetzung umfassend flüssigen Ammoniak aus Schritt (h) sowie gegebenenfalls der anderen Gasströme gemäß Schritt (i) vereint. Bevorzugt wird diese Verfahrensvariante im Betrieb der Harnstoff-Anlage im vollintegrierten Modus (Fall I) entweder in Teillast oder in Volllastangewendet.

In einer bevorzugten Ausführungsform umfasst das erfindungsgemäße Verfahren den zusätzlichen Schritt (I), bei dem zumindest ein Teil des komprimierten Kohlendioxidstroms aus Schritt (d) ausgeschleust wird.

In einer bevorzugten Ausführungsform des erfindungsgemäßen Verfahrens wird die Gesamtmenge des komprimierten Kohlendioxidstroms aus Schritt (d) ausgeschleust.

In einer weiteren bevorzugten Ausführungsform wird der komprimierte Kohlendioxidstrom vor dem Ausschleusen durch eine Turbine geleitet.

Noch bevorzugter werden das Wasser und das Ammoniak, die im komprimierten Kohlendioxidstrom enthalten sind, vor dem Ausschleusen abgetrennt und/oder zurückgewonnen. Dem Fachmann sind Verfahren hierfür bekannt.

In einer besonders bevorzugten Ausführungsform wird der komprimierte Kohlendioxidstrom an die Luft abgegeben, besonders bevorzugt nachdem der komprimierte Kohlendioxidstrom entspannt und gereinigt wurde. Dem Fachmann sind Verfahren hierfür bekannt.

Bei einem Betrieb nach Fall (II) (nicht zur Erfindung gehörend) findet nur eine Ammoniaksynthese, aber keine Harnstoffsynthese statt, wobei bevorzugt Schritt (i) nicht durchgeführt wird.

Besonders bevorzugt werden bei einer alleinigen Synthese von Ammoniak die Schritte (b), (e), (g), (i) und (k) nicht durchgeführt und in Schritt (f) wird nicht der vereinte Synthesegasstrom aus Schritt (e), sondern ein durch Methanisierung gemäß Schritt (i*) gebildeter Synthesegasstrom komprimiert, aus dem dann Ammoniak synthetisiert wird.

Gegenstand der vorliegenden Erfindung ist weiterhin eine Anlage zur Herstellung von Ammoniak und Harnstoff umfassend die folgenden miteinander in Wirkverbindung stehenden Komponenten:
eine Vorrichtung konfektioniert zur Bereitstellung eines Rohsynthesegasstroms umfassend Wasserstoff, Stickstoff und Kohlendioxid, sowie gegebenenfalls geringere Anteile an Methan, Argon, Kohlenmonoxid und evtl. Helium;
mindestens einen Verdichter konfektioniert zum Komprimieren des Rohsynthesegasstroms unter Bildung eines komprimierten Synthesegasstroms;
mindestens eine Waschkolonne konfektioniert zum Waschen des komprimierten Synthesegasstroms mit einer Zusammensetzung umfassend Ammoniak unter Bildung eines an Kohlendioxid abgereicherten gereinigten Synthesegasstroms und eines Kondensats;
mindestens einen ersten Reaktor konfektioniert zum Synthetisieren von Ammoniak aus dem gereinigten Synthesegasstrom;
mindestens einen zweiten Reaktor konfektioniert zum Synthetisieren von Harnstoff aus dem Kondensat unter Bildung einer wässrigen Harnstoffzusammensetzung,
wobei erfindungsgemäß die Anlage weiterhin mindestens eine in Strömungsrichtung dem Verdichter nachgeschaltete Vorrichtung aufweist, konfektioniert zum Aufteilen des komprimierten Synthesegasstroms aus dem Verdichter in einen ersten Synthesegasstrom und in einen zweiten Synthesegasstrom, wobei von der Aufteilvorrichtung eine erste Leitung zu der Waschkolonne führt, über die nur der erste Synthesegasstrom der Waschkolonne zuführbar ist, wobei die Anlage weiterhin einen Stripper umfasst und von der Aufteilvorrichtung eine zweite Leitung zu dem Stripper führt, über die nur der zweite Synthesegastrom dem Stripper zuführbar ist, wobei eine von dem zweiten Reaktor ausgehende Leitung zu dem Stripper vorgesehen ist, über die die wässrige Harnstoffzusammensetzung aus dem zweiten Reaktor dem Stripper zuführbar ist.
Bei der erfindungsgemäßen Anlage kann dann, wenn kein Bedarf an Harnstoff besteht, im gleichen Anlagenkomplex nur Ammoniak hergestellt werden. Hier unterscheidet sich die erfindungsgemäße Anlage von aus dem Stand der Technik bekannten Lösungen hinsichtlich ihrer Flexibilität. Die erfindungsgemäße Anlage kann zudem vorteilhaft nur in Teillast betrieben werden und es kann eine spezifische Betriebsweise in einer Start-up-Phase und/oder in einer Shut-down-Phase des Verfahrens vorgesehen sein.

Vorzugsweise umfasst die erfindungsgemäße Anlage weiterhin eine zweite, in Strömungsrichtung dem Verdichter vorgeschaltete Aufteilvorrichtung, mittels derer der Rohsynthesegasstrom aufteilbar ist in zwei Teilströme, wobei eine erste Leitung für einen ersten Teilstrom von der zweiten Aufteilvorrichtung zum Verdichter führt und eine zweite Leitung für einen dritten Teilstrom von der zweiten Aufteilvorrichtung zu einer Waschvorrichtung führt, welche konfektioniert ist zum Waschen des dritten Teilstroms des Rohsynthesegases mit einer Waschlösung umfassend Wasser und Ammoniak unter Bildung eines an Kohlendioxid abgereicherten vorgereinigten Synthesegasstroms und einer an Kohlendioxid angereicherten Waschlösung und zum Austreiben von Kohlendioxid aus der an Kohlendioxid angereicherten Waschlösung unter Bildung eines komprimierten Kohlendioxidstroms, wobei weiterhin Mittel vorgesehen sind zum Vereinen des vorgereinigten dritten Teilsynthesegasstroms aus der Waschvorrichtung stromabwärts der Waschvorrichtung und stromaufwärts des Kompressors mit dem ersten Teilsynthesegasstrom unter Bildung eines vereinten Synthesegasstroms, welcher über eine Leitung dem Verdichter zuführbar ist.

Eine bevorzugte Weiterbildung der Erfindung betrifft eine Anlage zur integrierten Herstellung von Ammoniak und Harnstoff, wobei die Anlage die folgenden miteinander in Wirkverbindung stehenden Komponenten umfasst:
(A) eine Vorrichtung konfektioniert zur Bereitstellung des Rohsynthesegasstroms umfassend Wasserstoff, Stickstoff und Kohlendioxid sowie gegebenenfalls geringere Anteile an Methan, Argon, Kohlenmonoxid und evtl. Helium;
(B) eine Vorrichtung konfektioniert zum Aufteilen des Rohsynthesegasstroms in einen ersten Rohsynthesegasstrom und in einen dritten Rohsynthesegasstrom;
(C) eine Vorrichtung konfektioniert zum Waschen des dritten Rohsynthesegasstroms aus (B) mit einer Waschlösung umfassend Wasser und Ammoniak unter Bildung eines an Kohlendioxid abgereicherten vorgereinigten Synthesegasstroms, einer an Kohlendioxid angereichten Waschlösung und eines komprimierten Kohlendioxidstroms;
(D) Mittel zum Vereinen des vorgereinigten dritten Synthesegasstroms aus (C) mit dem ersten Rohsynthesegasstrom aus (B) unter Bildung eines vereinten Synthesegasstroms;
(E) einen Verdichter konfektioniert zum Komprimieren eines Synthesegasstroms, beispielsweise des vereinten Synthesegasstroms aus (D), unter Bildung eines komprimierten Synthesegasstroms;
(F) eine Vorrichtung konfektioniert zum Aufteilen des komprimierten Synthesegasstroms aus (E) in einen ersten Synthesegasstrom und in einen zweiten Synthesegasstrom;
(G) eine Waschkolonne konfektioniert zum Waschen des ersten Synthesegasstroms aus (F) mit einer Zusammensetzung umfassend flüssigen Ammoniak unter Bildung eines an Kohlendioxid abgereicherten gereinigten Synthesegasstroms und eines Kondensats;
(H) einen Reaktor konfektioniert zum Synthetisieren von Ammoniak aus dem gereinigten Synthesegasstrom aus (G); und
(I) einen Reaktor konfektioniert zum Synthetisieren von Harnstoff aus dem Kondensat aus (G) unter Bildung einer wässrigen Harnstoffzusammensetzung.

Alle im Zusammenhang mit dem erfindungsgemäßen Verfahren beschriebenen bevorzugten Ausführungsformen gelten analog für die erfindungsgemäße Anlage und werden daher an dieser Stelle nicht wiederholt.

Die erfindungsgemäße Anlage umfasst eine Vorrichtung konfektioniert zum Bereitstellen eines Rohsynthesegasstroms. Bevorzugt umfasst diese einen Dampfreformer und/oder einen autothermen Reformer. Umfasst die Anlage einen autothermen Reformer, kann die Menge an CO₂, welche in dem autothermen Reformer gebildet wird, durch Variation von Reaktionsparametern wie beispielsweise dem Druck oder dem Dampf/Kohlenstoff-Verhältnis kontrolliert werden. Eine bevorzugte Ausführungsform der Anlage umfasst zusätzlich eine Vorrichtung konfektioniert zur Konvertierung von CO zu CO₂ und/oder eine Vorrichtung konfektioniert zur Wärmeintegration.

Die erfindungsgemäße Anlage umfasst vorzugsweise eine Vorrichtung konfektioniert zum Aufteilen des Rohsynthesegasstroms in einen ersten Rohsynthesegasstrom und in einen dritten Rohsynthesegasstrom. Bevorzugt ist diese Vorrichtung so konfektioniert, dass das Volumenverhältnis zwischen den Rohsynthesegasströmen in der Durchführung des erfindungsgemäßen Verfahrens variiert werden kann.

Die erfindungsgemäße Anlage umfasst vorzugsweise eine Vorrichtung konfektioniert zum Waschen des dritten Rohsynthesegasstroms aus (B) mit einer Waschlösung umfassend Wasser und Ammoniak unter Bildung eines an Kohlendioxid abgereicherten vorgereinigten Synthesegasstroms und einer an Kohlendioxid angereicherten Waschlösung und zum Austreiben von Kohlendioxid aus der an Kohlendioxid angereicherten Waschlösung unter Bildung eines komprimierten Kohlendioxidstroms.

Die erfindungsgemäße Anlage umfasst vorzugsweise ein Mittel zum Vereinen des vorgereinigten Synthesegasstroms aus (C) mit dem ersten Rohsynthesegasstrom aus (B) unter Bildung eines vereinten Synthesegasstroms.

Die erfindungsgemäße Anlage umfasst einen Verdichter konfektioniert zum Komprimieren eines Synthesegasstroms, beispielsweise des vereinten Synthesegasstroms aus (D), unter Bildung eines komprimierten Synthesegasstroms.

In einer bevorzugten Ausführungsform umfasst die Anlage keinen weiteren Verdichter. Besonders bevorzugt umfasst die Vorrichtung keinen in der konventionellen Harnstoffsynthese üblichen CO₂-Verdichter.

Die erfindungsgemäße Anlage umfasst eine Vorrichtung konfektioniert zum Aufteilen des komprimierten Synthesegasstroms aus (E) in einen ersten Synthesegasstrom und in einen zweiten Synthesegasstrom.

Die erfindungsgemäße Anlage umfasst eine Waschkolonne konfektioniert zum Waschen des ersten Synthesegasstroms aus (F) mit einer Zusammensetzung umfassend flüssigen Ammoniak unter Bildung eines an Kohlendioxid abgereicherten gereinigten Synthesegasstroms und eines Kondensats.

Die erfindungsgemäße Anlage umfasst einen Reaktor konfektioniert zum Synthetisieren von Ammoniak aus dem gereinigten Synthesegasstrom aus (G).

Die erfindungsgemäße Anlage umfasst einen Reaktor konfektioniert zum Synthetisieren von Harnstoff aus dem Kondensat aus (G) unter Bildung einer wässrigen Harnstoffzusammensetzung.

Bevorzugt umfasst die erfindungsgemäße Anlage keine Vorrichtung zur konventionellen CO₂-Wäsche, wie sie in konventionellen Anlagen zur Synthese von NH₃ häufig nach der CO Konvertierung eingesetzt werden, sowie keinen ansonsten für die Harnstoffsynthese zusätzlich erforderlichen CO₂-Verdichter samt seiner gesamten Peripherie.

In einer bevorzugten Ausführungsform umfasst die erfindungsgemäße Anlage zusätzlich die folgenden Komponenten:
- eine Vorrichtung (J) konfektioniert zum Strippen der wässrigen Harnstoffzusammensetzung aus (I) mit dem zweiten Synthesegasstrom aus (F) unter Bildung eines Strippgasstroms und einer gereinigten wässrigen Harnstofflösung;
- Mittel (K) zum Vereinen des Strippgasstroms aus (J) mit der Zusammensetzung umfassend flüssigen Ammoniak aus (G); und/oder
- eine Vorrichtung (L) konfektioniert zum Methanisieren des an Kohlendioxid abgereicherten gereinigten Synthesegasstroms aus (G) unter Bildung eines Reinstsynthesegasstroms.

In einer weiteren bevorzugten Ausführungsform umfasst die erfindungsgemäße Anlage zusätzlich die folgenden Komponenten:
- Mittel (M) zum Vereinen zumindest eines Teils des komprimierten Kohlendioxidstroms aus (C) mit der Zusammensetzung umfassend flüssigen Ammoniak aus (G);
- Mittel (N) zum Vereinen zumindest eines Teils des Ammoniaks aus (H) mit der Zusammensetzung umfassend flüssigen Ammoniak aus (G); und/oder
- eine Vorrichtung (O) konfektioniert zum Ausschleusen zumindest eines Teils des komprimierten Kohlendioxidstroms aus Schritt (C).

Bevorzugt umfasst die Vorrichtung (O) eine Turbine, durch die der komprimierte Kohlendioxidstrom vor dem Ausschleusen geleitet wird. Noch bevorzugter umfasst die Vorrichtung (O) auch eine Vorrichtung konfektioniert zum Abtrennen und/oder Zurückgewinnen des Wassers und des Ammoniaks, die im komprimierten Kohlendioxidstrom enthalten sind, vor dem Ausschleusen. Eine weitere bevorzugte Ausführungsform von Vorrichtung (O) umfasst eine Vorrichtung konfektioniert zum Abgeben des Kohlendioxids an die Luft.

Alle im Zusammenhang mit dem erfindungsgemäßen Verfahren beschriebenen bevorzugten Ausführungsformen gelten analog für die bevorzugten Ausführungsformen der Anlage und werden daher an dieser Stelle nicht wiederholt.

In einer nicht zur Erfindung gehörenden Ausführungsform des Konzepts wird nur die Ammoniak-Anlage ohne gleichzeitigen Betrieb der Harnstoff-Anlage betrieben (Fall II). In diesem Fall wird das Kohlendioxid bevorzugt - wie bei der konventionellen Prozessführung - an die Luft oder alternativ an eine weitere benachbarte Harnstoff-Anlage abgegeben.

Bei der nicht zur Erfindung gehörenden alleinigen Ammoniak-Synthese (Fall II) mit der erfindungsgemäßen Anlage umfasst das Verfahren erfindungsgemäß die folgenden Schritte:
- Bereitstellen eines Rohsynthesegasstroms umfassend Wasserstoff, Stickstoff und Kohlendioxid sowie ggf. geringere Anteile an Methan, Argon, Kohlenmonoxid und evtl. Helium;
- Waschen des Rohsynthesegasstroms mit einer Waschlösung umfassend Wasser und Ammoniak unter Bildung eines an Kohlendioxid abgereicherten vorgereinigten Synthesegasstroms und einer an Kohlendioxid angereicherten Waschlösung;
- Austreiben von Kohlendioxid aus der an Kohlendioxid angereicherten Waschlösung unter Bildung eines komprimierten Kohlendioxidstroms;
- Methanisieren des vorgereinigten Synthesegasstroms unter Bildung eines Reinstsynthesegasstroms;
- Überführen des Reinstsynthesegasstroms in einen Verdichter konfektioniert zum Komprimieren des vereinten Synthesegasstroms unter Bildung eines komprimierten Reinstsynthesegasstroms;
- Überführen des komprimierten Reinstsynthesegasstroms in den Reaktor konfektioniert zum Synthetisieren von Ammoniak; und
- Synthetisieren von Ammoniak.

Die erfindungsgemäße Anlage eignet sich besonders zum Durchführen des erfindungsgemäßen Verfahrens. Ein weiterer Aspekt der Erfindung betrifft daher die Verwendung der erfindungsgemäßen Anlage in dem erfindungsgemäßen Verfahren.

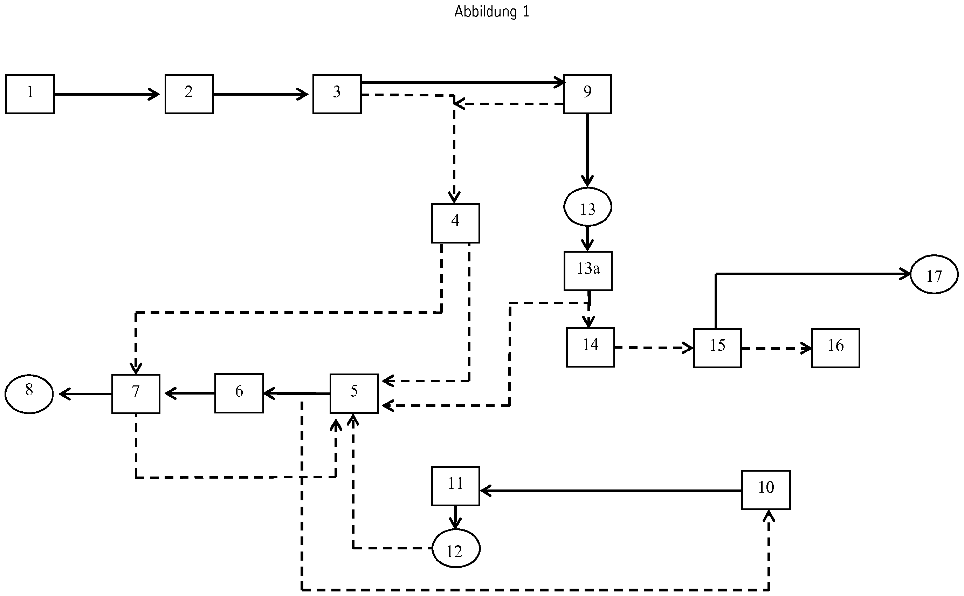

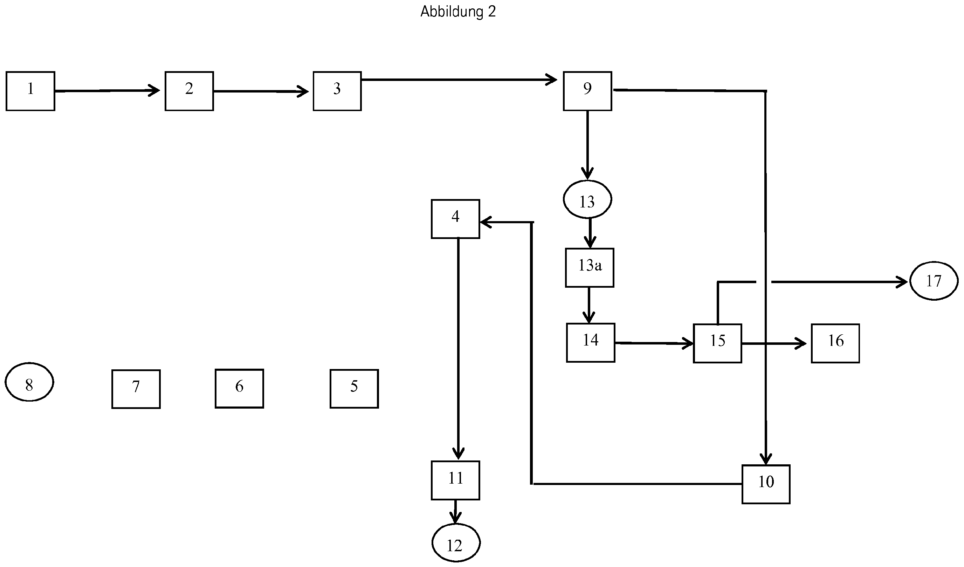

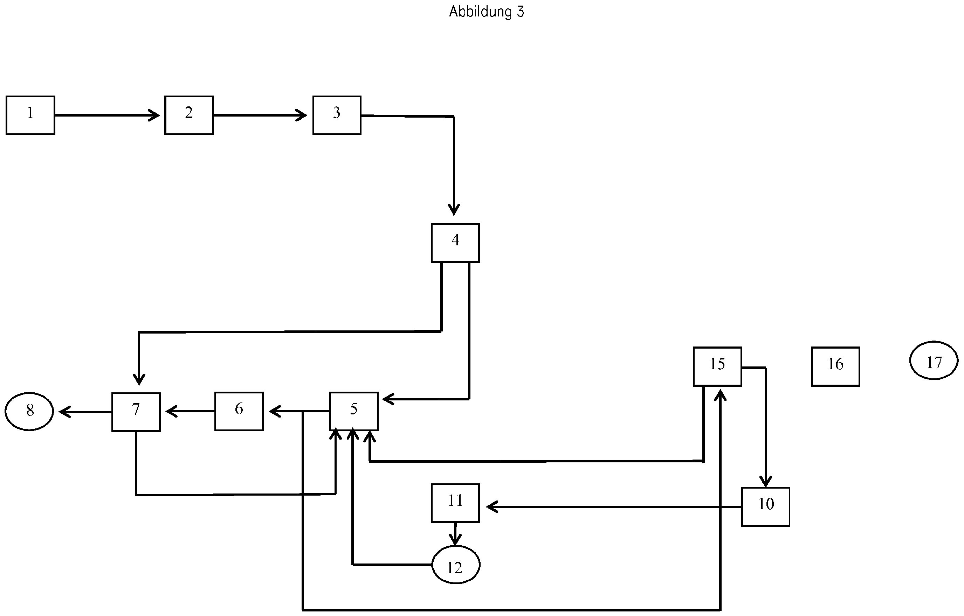

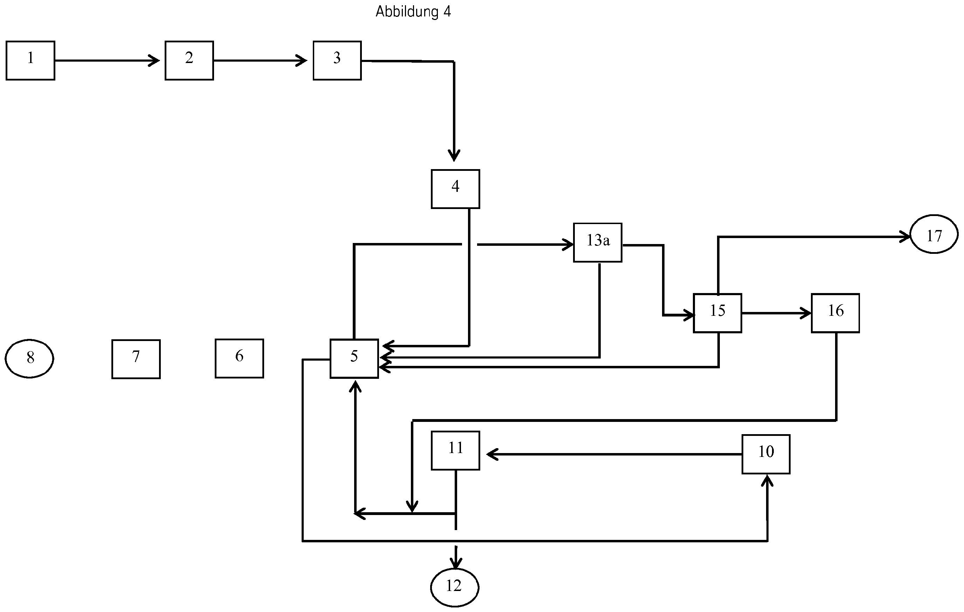

Die Erfindung und ihre bevorzugten Ausführungsformen werden nachfolgend anhand der Verfahrensschemata in den Abbildungen 1 und 3 erläutert. Abbildungen 2 und 4 zeigen nicht zur Erfindung gehörende Ausführungsformen.

Abbildung 1 illustriert schematisch eine bevorzugte Variante des erfindungsgemäßen Verfahrens, bei dem die Ammoniak- und die Harnstoff-Anlage im vollintegrierten Voll- oder Teillast-Modus (Fall I) betrieben werden. Dabei deuten gestrichelte Linien Stoffströme an, welche erfindungsgemäß variabel sind, d.h. bei bestimmten Betriebsweisen verschwinden können.

Das Rohsynthesegas wird bereitgestellt, nachdem es bevorzugt einen Reformer (1), und/oder bevorzugt eine CO-Konvertierung (2) und/oder bevorzugt eine Wärmeintegration (3) durchlaufen hat. Anschließend wird das Rohsynthesegas gegebenenfalls in einen ersten und in einen dritten Rohsynthesegasstrom aufgeteilt, die bevorzugt im zeitlichen Ablauf des Verfahrens unterschiedlich groß sein können. Der dritte Rohsynthesegasstrom wird gegebenenfalls in einer Vorrichtung (9) mit einer Waschlösung umfassend Wasser und Ammoniak gewaschen. Dabei werden ein an Kohlendioxid vorgereinigter Synthesegasstrom und eine an Kohlendioxid angereichte Waschlösung (15) gebildet.

Aus dieser Waschlösung wird das Kohlendioxid in der Vorrichtung (13a) unter Bildung eines komprimierten Kohlendioxidstroms gegebenenfalls ausgetrieben und zwar auf einen Druck, der leicht oberhalb des Niveaus der Harnstoffsynthese liegt und hoch genug ist, um die Druckverluste bis zur eigentlichen Harnstoffsynthese zu kompensieren.

Der vorgereinigte Synthesegasstrom wird gegebenenfalls mit dem ersten Rohsynthesegasstrom vereint. Der so gegebenenfalls gebildete vereinte Synthesegasstrom wird in einem Synthesegaskompressor (4) auf einen Druck komprimiert, der leicht oberhalb des Niveaus der Harnstoffsynthese liegt und hoch genug ist, um die Druckverluste bis zur eigentlichen Harnstoffsynthese zu kompensieren.

Dann wird der auf diese Weise gebildete komprimierte Synthesegasstrom in einen ersten und einen zweiten Synthesegasstrom geteilt. Für das nachfolgende Waschen des ersten Synthesegasstroms in einer Waschkolonne (5) mit einer Zusammensetzung umfassend flüssiges Ammoniak wird flüssiges Ammoniak (12) bereitgestellt.

Bevorzugt wird das flüssige Ammoniak ganz aus der Ammoniaksynthese (11) bereitgestellt. Auch aus einem Stripper (7) kann bevorzugt ein Teil der Zusammensetzung bereitgestellt werden. Beim Waschen des ersten Synthesegasstroms in der Waschkolonne (5) mit einer Zusammensetzung umfassend flüssiges Ammoniak werden ein an Kohlendioxid abgereicherter gereinigter Synthesegasstrom und ein Kondensat gebildet. Bevorzugt kann auch der komprimierte Kohlendioxidstrom ganz oder teilweise in die Waschkolonne (5) überführt und dort umgesetzt werden.

Für die Ammoniaksynthese wird dann der an Kohlendioxid abgereicherte gereinigte Synthesegasstrom in einen Ammoniak-Reaktor (11) zur Synthese geleitet. Bevorzugt kann aus dem gereinigten Synthesegasstrom vorher in einer zur Methanisierung konfektionierten Vorrichtung (10) ein Reinstsynthesegasstrom gebildet werden, der dann in den Ammoniak-Reaktor (11) zur Synthese überführt wird.

Für die Harnstoffsynthese wird das Kondensat aus der Waschkolonne (5) in einen Harnstoff-Reaktor (6) überführt und eine wässrige Harnstoffzusammensetzung gebildet. Bevorzugt werden aus dieser Harnstoffzusammensetzung in einem Stripper (7) ein Stripgasstrom und eine gereinigte wässrige Harnstofflösung gebildet, welche dann bevorzugt durch weitere, dem Fachmann bekannte Prozessschritte in ein Harnstoffgranulat (8) überführt wird.

In einer bevorzugten Ausführungsform werden der zweite Synthesegasstrom und die Harnstoffzusammensetzung in den Stripper (7) geleitet. In einer weiteren bevorzugten Ausführungsform wird der Teil des Kohlendioxidstroms auf hohem Druck, der nicht zur Waschkolonne (5) geleitet wird, durch eine Turbine (14) geleitet, danach durch eine Vorrichtung (15), konfektioniert zur Ammoniak-Abtrennung durch Wasser-Absorption, geführt und anschließend als gereinigter Kohlendioxidstrom (17) an die Umgebung abgegeben. Das Ammoniak in der mit Ammoniak beladenen wässrigen Lösung aus der Ammoniak-Abtrennung (15) wird in einer Vorrichtung (16), konfektioniert zur Ammoniak-Rückgewinnung, durch Desorption aus der wässrigen Lösung ausgeschleust und in den Prozess an geeigneter Stelle zurückgeführt.

Abbildung 2 illustriert schematisch eine bevorzugte Variante des nicht zur Erfindung gehörenden Verfahrens, bei der mit der erfindungsgemäßen Vorrichtung nur Ammoniak (Fall II) synthetisiert wird.

Der Rohsynthesegasstrom wird im Unterschied zu Abbildung 1 (Fall I) nicht aufgeteilt. Der Rohsynthesegasstrom wird in einer Vorrichtung (9) mit einer Waschlösung umfassend Wasser und Ammoniak gewaschen. Dabei werden ein an Kohlendioxid vorgereinigter Synthesegasstrom und eine an Kohlendioxid angereicherte Waschlösung (15) gebildet. Aus dieser Waschlösung wird das Kohlendioxid unter Bildung eines komprimierten Kohlendioxidstroms in der Vorrichtung (13a) ausgetrieben.

Der komprimierte Kohlendioxidstrom wird nach der Desorption (13a) durch eine Turbine (14) geleitet, danach durch eine Vorrichtung (15), konfektioniert zur Ammoniak-Abtrennung durch Wasser-Absorption, geführt und anschließend als gereinigter Kohlendioxidstrom (17) an die Umgebung abgegeben. Das Ammoniak in der mit Ammoniak beladenen wässrigen Lösung aus der Ammoniak-Abtrennung (15) wird in einer Vorrichtung (16), konfektioniert zur Ammoniak-Rückgewinnung, durch Desorption aus der wässrigen Lösung ausgeschleust und in den Prozess zurückgeführt. Der vorgereinigte Synthesegasstrom aus (9) wird in eine zur Methanisierung konfektionierte Vorrichtung (10) überführt und ein Reinstsynthesegasstrom gebildet. In einem Synthesegaskompressor (4) wird der Reinstsynthesegasstrom komprimiert und dann in den Ammoniak-Reaktor (11) zur Ammoniak-Synthese überführt.

Abbildung 3 illustriert schematisch eine weitere bevorzugte Variante des erfindungsgemäßen Verfahrens, bei dem die Ammoniak- und die Harnstoff-Anlage in einem alternativen vollintegrierten Modus (Fall III) betrieben werden.

Das Rohsynthesegas wird bereitgestellt, nachdem es bevorzugt einen Reformer (1), und/oder bevorzugt eine CO-Konvertierung (2) und/oder bevorzugt eine Wärmeintegration (5) durchlaufen hat. Im Unterschied zu Abbildung 1 (Fall I) wird das gesamte Rohsynthesegas nun in einem Synthesegaskompressor (4) auf einen Druck komprimiert, der leicht oberhalb des Niveaus der Harnstoffsynthese liegt und hoch genug ist, um die Druckverluste bis zur eigentlichen Harnstoffsynthese zu kompensieren. Der komprimierte Rohsynthesegasstrom wird geteilt und ein zweiter Teil in einen Stripper (7) überführt.

Der erste Teil wird in die Waschkolonne (5) mit einer Zusammensetzung umfassend flüssigen Ammoniak überführt. Das flüssige Ammoniak dieser Zusammensetzung wird dabei ganz aus der Ammoniaksynthese (11) bereitgestellt. Auch das gasförmige Produkt aus dem Stripper (7) wird komplett in die Waschkolonne (5) überführt und als Teil der Zusammensetzung der Waschkolonne (5) bereitgestellt. Beim Waschen des ersten Teils des Rohsynthesegasstroms und des gasförmigen Produktes aus dem Stripper (7) in der Waschkolonne (5) werden ein an Kohlendioxid abgereicherter gereinigter Synthesegasstrom und ein Kondensat gebildet. Für die Harnstoffsynthese wird das Kondensat in einen Harnstoff-Reaktor (6) überführt und eine wässrige Harnstoffzusammensetzung gebildet. Aus dieser Harnstoffzusammensetzung werden dann in einem Stripper (7) ein Strippgasstrom und eine gereinigte wässrige Harnstofflösung gebildet.

Der gereinigte Synthesegasstrom wird in eine Vorrichtung (15) überführt, die zur Ammoniak-Abtrennung und Wasser-Absorption konfektioniert ist. Der dort abgetrennte Ammoniak wird ebenfalls in die Waschkolonne (5) zurückgeführt. Aus dem verbleibenden Gas wird in einer zur Methanisierung konfektionierten Vorrichtung (10) ein Reinstsynthesegasstrom gebildet, der dann in den Ammoniak-Reaktor (11) zur Ammoniak-Synthese überführt wird.

Abbildung 4 illustriert schematisch eine nicht zur Erfindung gehörende Variante des Verfahrens, bei der mit der erfindungsgemäßen Vorrichtung nur Ammoniak in einem alternativen vollintegrierten Modus (Fall IV) synthetisiert wird.

Der Rohsynthesegasstrom wird wie in Abbildung 3 nicht aufgeteilt und in einem Synthesegaskompressor (4) zunächst auf einen Druck komprimiert, der leicht oberhalb des Niveaus der Harnstoffsynthese liegt und hoch genug ist, um die Druckverluste bis zur eigentlichen Harnstoffsynthese zu kompensieren. Abweichend zu der in Abbildung 3 dargestellten Betriebsweise nach Fall III wird der komprimierte Rohsynthesegasstrom nicht geteilt, sondern in die Waschkolonne (5) überführt. In der Waschkolonne (5) wird der Rohsynthesegasstrom mit einer Zusammensetzung umfassend flüssiges Ammoniak in Kontakt gebracht und ein an Kohlendioxid abgereicherter gereinigter Synthesegasstrom und ein Kondensat gebildet. Das flüssige Ammoniak (12) dieser Zusammensetzung wird dabei ganz aus der Ammoniaksynthese (11) bereitgestellt.

Der gereinigte Synthesegasstrom wird in eine zur Methanisierung konfektionierte Vorrichtung (10) überführt und ein Reinstsynthesegasstrom gebildet. Der Reinstsynthesegasstrom wird dann in den Ammoniak-Reaktor (11) zur Ammoniak-Synthese überführt und Ammoniak (12) synthetisiert.

Das an Kohlendioxid angereicherte Kondensat aus der Waschkolonne (5) wird in eine Desorption (13a) geleitet und ein gasförmiger Kohlendioxidstrom sowie ein an Kohlendioxid abgereichertes Kondensat gewonnen. Das an Kohlendioxid abgereicherte Kondensat wird wieder in die Waschkolonne (5) zurückgeführt. Der Kohlendioxidstrom wird in eine Vorrichtung (15) überführt, in der das noch im Kohlendioxidstrom enthaltene Ammoniak, z.B. durch Absorption in Wasser, abgetrennt wird, bevor der gereinigte Kohlendioxidstrom (17) an die Umgebung abgegeben werden kann. Das abgetrennte Ammoniak wird in der Vorrichtung (16), konfektioniert zur Ammoniak-Rückgewinnung, durch Desorption aus der wässrigen Lösung ausgeschleust und in die Waschkolonne (5) zurückgeführt.

### Bezugszeichenliste:

- 1: Reformer
- 2: CO-Konvertierung
- 3: Wärmeintegration
- 4: Synthesegaskompressor
- 5: Waschvorrichtung/Waschkolonne mit einer Zusammensetzung umfassend flüssigen Ammoniak
- 6: Harnstoff-Reaktor
- 7: Stripper
- 8: Harnstoff
- 9: Vorrichtung mit einer Waschlösung umfassend Wasser und Ammoniak
- 10: Methanisierung
- 11: Ammoniak-Reaktor
- 12: Ammoniak
- 15: An Kohlendioxid angereichte Waschlösung auf hohem Druck
- 13a: Desorption
- 14: Turbine
- 15: Ammoniak Abtrennung / Wasser Absorption
- 16: Ammoniak Rückgewinnung / Wasser Desorption
- 17: Ausschleusung von Kohlendioxid

## Patentansprüche

1. Verfahren zur Herstellung von Ammoniak und Harnstoff in einem gemeinsamen Anlagenkomplex, wobei ein Rohsynthesegasstrom, welcher mindestens die Gase Wasserstoff, Stickstoff und Kohlendioxid enthält, zunächst komprimiert wird, danach ein Teilstrom des Rohsynthesegases mit Ammoniak gewaschen wird, wobei ein an Kohlendioxid abgereicherter gereinigter Synthesegasstrom gebildet wird sowie ein Kondensat, wobei aus dem an Kohlendioxid abgereicherten gereinigten Synthesegasstrom Ammoniak synthetisiert wird und aus dem Kondensat Harnstoff synthetisiert wird unter Bildung einer wässrigen Harnstoffzusammensetzung,
**dadurch gekennzeichnet, dass** bei Herstellung von Ammoniak und Harnstoff der Rohsynthesegasstrom nach der Komprimierung in zwei Teilströme aufgeteilt wird, nämlich einen ersten Synthesegasteilstrom und einen zweiten Synthesegasteilstrom, wobei nur der erste Synthesegasteilstrom mit flüssigem Ammoniak gewaschen wird.

2. Verfahren nach Anspruch 1, **dadurch gekennzeichnet, dass** der zweite Synthesegasteilstrom nach dem Komprimieren einem Stripper (7) zugeführt wird und in dem Stripper (7) zum Strippen der wässrigen Harnstoffzusammensetzung verwendet wird unter Bildung eines Stripgasstroms und einer gereinigten wässrigen Harnstofflösung.

3. Verfahren nach Anspruch 1 oder 2, **dadurch gekennzeichnet, dass** der Rohsynthesegasstrom bereits vor der Komprimierung in zwei Teilströme aufgeteilt wird, unter Bildung eines dritten Synthesegasteilstroms und eines Rohsynthesegasstroms, welcher einem Kompressor (4) zugeführt wird, wobei der dritte Synthesegasteilstrom in einer Waschvorrichtung (9) mit einer Waschlösung umfassend Wasser und Ammoniak gewaschen wird unter Bildung eines an Kohlendioxid abgereicherten vorgereinigten Synthesegasstroms und einer an Kohlendioxid angereicherten Waschlösung (15), wobei der vorgereinigte Synthesegasstrom danach wieder mit dem Rohsynthesegasstrom vereinigt und mit diesem gemeinsam dem Kompressor (4) zugeführt wird.

4. Verfahren nach Anspruch 3, **dadurch gekennzeichnet, dass** aus der Waschlösung (15) in einer Desorptionsvorrichtung (13 a) Kohlendioxid ausgetrieben wird und zwar auf einen Druck, der oberhalb des Niveaus der Harnstoffsynthese liegt.

5. Verfahren nach Anspruch 4, **dadurch gekennzeichnet, dass** mindestens ein Teil des komprimierten Kohlendioxids der Wäsche (5) mit flüssigem Ammoniak zugeführt wird und mindestens ein Teil des komprimierten Kohlendioxids aus dem Anlagenkomplex ausgeschleust wird oder die Gesamtmenge des komprimierten Kohlendioxids aus dem Anlagenkomplex ausgeschleust wird oder die Gesamtmenge des komprimierten Kohlendioxids der Wäsche (5) zugeführt wird.

6. Verfahren nach einem der Ansprüche 4 oder 5, **dadurch gekennzeichnet, dass** mindestens ein Teil des komprimierten Kohlendioxids durch eine Turbine (14) geleitet wird und in einer Vorrichtung zur Ammoniakabtrennung (15) Ammoniak abgetrennt und das gereinigte Kohlendioxid (17) an die Umgebung abgegeben wird.

7. Anlage zur Herstellung von Ammoniak und Harnstoff umfassend die folgenden miteinander in Wirkverbindung stehenden Komponenten:
eine Vorrichtung (1) konfektioniert zur Bereitstellung eines Rohsynthesegasstroms umfassend Wasserstoff, Stickstoff und Kohlendioxid, sowie gegebenenfalls geringere Anteile an Methan, Argon, Kohlenmonoxid und evtl. Helium;
mindestens einen Verdichter (4) konfektioniert zum Komprimieren des Rohsynthesegasstroms unter Bildung eines komprimierten Synthesegasstroms;
mindestens eine Waschkolonne (5) konfektioniert zum Waschen des komprimierten Synthesegasstroms mit einer Zusammensetzung umfassend Ammoniak unter Bildung eines an Kohlendioxid abgereicherten gereinigten Synthesegasstroms und eines Kondensats;
mindestens einen ersten Reaktor (11) konfektioniert zum Synthetisieren von Ammoniak aus dem gereinigten Synthesegasstrom;
mindestens einen zweiten Reaktor (6) konfektioniert zum Synthetisieren von Harnstoff aus dem Kondensat unter Bildung einer wässrigen Harnstoffzusammensetzung,
**dadurch gekennzeichnet, dass** die Anlage weiterhin mindestens eine in Strömungsrichtung dem Verdichter (4) nachgeschaltete Vorrichtung aufweist, konfektioniert zum Aufteilen des komprimierten Synthesegasstroms aus dem Verdichter (4) in einen ersten Synthesegasstrom und in einen zweiten Synthesegasstrom, wobei von der Aufteilvorrichtung eine erste Leitung zu der Waschkolonne (5) führt, über die nur der erste Synthesegasstrom der Waschkolonne (5) zuführbar ist, wobei die Anlage weiterhin einen Stripper (7) umfasst und von der Aufteilvorrichtung eine zweite Leitung zu dem Stripper (7) führt, über die nur der zweite Synthesegastrom dem Stripper (7) zuführbar ist, wobei eine von dem zweiten Reaktor (6) ausgehende Leitung zu dem Stripper (7) vorgesehen ist, über die die wässrige Harnstoffzusammensetzung aus dem zweiten Reaktor (6) dem Stripper zuführbar ist.

8. Anlage nach Anspruch 7, **dadurch gekennzeichnet, dass** diese weiterhin eine zweite, in Strömungsrichtung dem Verdichter (4) vorgeschaltete Aufteilvorrichtung umfasst, mittels derer der Rohsynthesegasstrom aufteilbar ist in zwei Teilströme, wobei eine erste Leitung für einen ersten Teilstrom von der zweiten Aufteilvorrichtung zum Verdichter führt und eine zweite Leitung für einen dritten Teilstrom von der zweiten Aufteilvorrichtung zu einer Waschvorrichtung (9) führt, welche konfektioniert ist zum Waschen des dritten Teilstroms des Rohsynthesegases mit einer Waschlösung umfassend Wasser und Ammoniak unter Bildung eines an Kohlendioxid abgereicherten vorgereinigten Synthesegasstroms und einer an Kohlendioxid angereicherten Waschlösung und zum Austreiben von Kohlendioxid aus der an Kohlendioxid angereicherten Waschlösung unter Bildung eines komprimierten Kohlendioxidstroms, wobei weiterhin Mittel vorgesehen sind zum Vereinen des vorgereinigten dritten Teilsynthesegasstroms aus der Waschvorrichtung (9) stromabwärts der Waschvorrichtung (9) und stromaufwärts des Kompressors mit dem ersten Teilsynthesegasstrom unter Bildung eines vereinten Synthesegasstroms, welcher über eine Leitung dem Verdichter (4) zuführbar ist.

9. Anlage nach Anspruch 7 oder 8 , **dadurch gekennzeichnet, dass** diese zusätzlich die folgenden Komponenten umfasst:
mindestens eine Leitung zum Zuführen eines Strippgasstroms aus dem Stripper (7) zu der Waschkolonne (5);
und/oder eine dem ersten Reaktor (11) zum Synthetisieren von Ammoniak im Strömungsweg vorgeschaltete Vorrichtung (10) konfektioniert zum Methanisieren (10) eines an Kohlendioxid abgereicherten gereinigten Synthesegasstroms aus der Waschkolonne (5) unter Bildung eines Reinstsynthesegasstroms.

10. Anlage nach einem der Ansprüche 8 oder 9, **dadurch gekennzeichnet, dass** diese zusätzlich eine Reihe von Vorrichtungen (14) bis (16) umfasst, konfektioniert zum Ausschleusen und zur Abtrennung und Rückgewinnung von Ammoniak und Wasser aus zumindest einem Teil des stromabwärts der Waschvorrichtung (9) gewonnenen komprimierten Kohlendioxidstroms.

11. Verwendung einer Anlage nach einem der Ansprüche 7 bis 10 in einem Verfahren nach einem der Ansprüche 1 bis 6.

## Claims

1. A process for preparing ammonia and urea in a joint facility, where a crude synthesis gas stream which contains at least the gases hydrogen, nitrogen and carbon dioxide is firstly compressed, then one substream of the crude synthesis gas is washed with ammonia, forming a purified synthesis gas stream depleted in carbon dioxide and also a condensate, where ammonia is synthesized from the purified synthesis gas stream which is depleted in carbon dioxide and urea is synthesized from the condensate to form an aqueous urea composition,
**characterized in that**, in the preparation of ammonia and urea, the crude synthesis gas stream is compressed and then divided into two substreams, namely a first synthesis gas substream and a second synthesis gas substream, with only the first synthesis gas substream being scrubbed with liquid ammonia.

2. The process as claimed in claim 1, **characterized in that** the second synthesis gas substream is, after compression, fed to a stripper (7) and used in the stripper (7) for stripping the aqueous urea composition to form a stripping gas stream and a purified aqueous urea solution.

3. The process as claimed in claim 1 or 2, **characterized in that** the crude synthesis gas stream is divided before compression into two substreams to form a third synthesis gas substream and a crude synthesis gas stream which is fed to a compressor (4), where the third synthesis gas stream is scrubbed with a scrubbing solution comprising water and ammonia in a scrubbing apparatus (9) to form a prepurified synthesis gas stream depleted in carbon dioxide and a scrubbing solution (13) enriched in carbon dioxide and the prepurified synthesis gas stream is then combined again with the crude synthesis gas stream and fed together with the latter to the compressor (4).

4. The process as claimed in claim 3, **characterized in that** carbon dioxide is driven off from the scrubbing solution (13) in a desorption apparatus (13a), and this is done at a pressure which is above the level of the urea synthesis.

5. The process as claimed in claim 4, **characterized in that** at least part of the compressed carbon dioxide is fed to the scrub (5) with liquid ammonia and at least part of the compressed carbon dioxide is discharged from the facility or the total amount of the compressed carbon dioxide is discharged from the facility or the total amount of the compressed carbon dioxide is fed to the scrub (5).

6. The process as claimed in either claim 4 or 5, **characterized in that** at least part of the compressed carbon dioxide is conveyed through a turbine (14) and ammonia is separated off in an apparatus for separating off ammonia (15) and the purified carbon dioxide (17) is released into the environment.

7. A plant for preparing ammonia and urea or alternatively only ammonia, which comprises the following components which are in active communication with one another:
an apparatus (1) configured for providing a crude synthesis gas stream comprising hydrogen, nitrogen and carbon dioxide and optionally relatively small proportions of methane, argon, carbon monoxide and possibly helium;
at least one compressor (4) configured for compressing the crude synthesis gas stream to form a compressed synthesis gas stream;
at least one scrubbing column (5) configured for scrubbing the compressed synthesis gas stream with a composition comprising ammonia to form a purified synthesis gas stream deleted in carbon dioxide and a condensate;
at least one first reactor (11) configured for synthesizing ammonia from the purified synthesis gas stream;
at least one second reactor (6) configured for synthesizing urea from the condensate to form an aqueous urea composition,
**characterized in that** according to the invention, the plant further comprises at least one apparatus which is located downstream of the compressor (4) in the flow direction and is configured for dividing the compressed synthesis gas stream from the compressor (4) into a first synthesis gas stream and a second synthesis gas stream, where a first conduit via which only the first synthesis gas stream can be fed to the scrubbing column (5) leads from the dividing apparatus to the scrubbing column (5), where the plant further comprises a stripper (7) and a second conduit via which only the second synthesis gas stream can be fed to the stripper (7) leads from the dividing apparatus to the stripper (7), where a conduit going from the second reactor to the stripper (7), via which the aqueous urea composition can be conveyed from the second reactor (6) to the stripper, is provided.

8. The plant as claimed in claim 7, **characterized in that** it further comprises a second dividing apparatus which is located upstream of the compressor (4) in the flow direction and by means of which the crude synthesis gas stream can be divided into two substreams, with a first conduit for a first substream leaving from the second dividing apparatus to the compressor and a second conduit for a third substream leading from the second dividing apparatus to a scrubbing apparatus (9) which is configured for scrubbing the third substream of the crude synthesis gas with a scrubbing solution comprising water and ammonia to form a prepurified synthesis gas stream depleted in carbon dioxide and a scrubbing solution enriched in carbon dioxide and for driving off carbon dioxide from the scrubbing solution enriched in carbon dioxide to form a compressed carbon dioxide stream, where means for combining the prepurified third synthesis gas substream from the scrubbing apparatus (9) downstream of the scrubbing apparatus (9) and upstream of the compressor with the first synthesis gas substream to form a combined synthesis gas stream which can be fed via a conduit to the compressor (4) are provided.

9. The plant as claimed in claim 7 or 8, **characterized in that** it additionally comprises the following components:
at least one conduit for feeding a stripping gas stream from the stripper (7) to the scrubbing column (5);
and/or an apparatus (10) which is located upstream of the first reactor (11) for the synthesis of ammonia and is configured for effecting methanation (10) of a purified synthesis gas stream depleted in carbon dioxide from the scrubbing column (5) to form a high-purity synthesis gas stream.

10. The plant as claimed in one of claims 8 or 9, **characterized in that** it additionally comprises a series of apparatuses (14) to (16) configured for removing and for separating off and recovering ammonia and water from at least part of the compressed carbon oxide stream obtained downstream of the scrubbing apparatus (9).

11. The use of a plant as claimed in any of claims 7 to 10 in a process as claimed in any of claims 1 to 6.

## Revendications

1. Procédé pour la préparation d'ammoniac et d'urée dans un complexe commun d'installations, un flux gazeux brut de synthèse, qui contient au moins les gaz hydrogène, azote et dioxyde de carbone, étant d'abord comprimé, ensuite un flux partiel du gaz brut de synthèse étant lavé avec de l'ammoniac, un flux gazeux de synthèse purifié, appauvri en dioxyde de carbone étant formé ainsi qu'un condensat, de l'ammoniac étant synthétisé à partir du flux gazeux de synthèse purifié, appauvri en dioxyde de carbone et de l'urée étant synthétisée à partir du condensat avec formation d'une composition aqueuse d'urée, **caractérisé en ce que** lors de la préparation d'ammoniac et d'urée, le flux gazeux brut de synthèse est réparti, après la compression, en deux flux partiels, à savoir un premier flux partiel gazeux de synthèse et un deuxième flux partiel gazeux de synthèse, seul le premier flux partiel gazeux de synthèse étant lavé avec de l'ammoniac liquide.

2. Procédé selon la revendication 1, **caractérisé en ce que** le deuxième flux partiel gazeux de synthèse est introduit, après la compression, dans un rectificateur (7) et utilisé dans le rectificateur (7) pour la rectification de la composition aqueuse d'urée avec formation d'un flux gazeux de rectification et d'une solution aqueuse purifiée d'urée.

3. Procédé selon la revendication 1 ou 2, **caractérisé en ce que** le flux gazeux brut de synthèse est déjà réparti en deux flux partiels avant la compression, avec formation d'un troisième flux partiel gazeux de synthèse et d'un flux gazeux brut de synthèse, qui est introduit dans un compresseur (4), le troisième flux partiel gazeux de synthèse étant lavé dans un dispositif de lavage (9) à l'aide d'une solution de lavage comprenant de l'eau et de l'ammoniac avec formation d'un flux gazeux de synthèse prépurifié, appauvri en dioxyde de carbone et d'une solution de lavage (13) enrichie en dioxyde de carbone, le flux gazeux de synthèse prépurifié étant ensuite de nouveau rassemblé avec le flux gazeux brut de synthèse et introduit conjointement avec celui-ci dans le compresseur (4).

4. Procédé selon la revendication 3, **caractérisé en ce que** du dioxyde de carbone est éliminé de la solution de lavage (13) dans un dispositif de désorption (13 a) et ce à une pression qui est supérieure à celle du niveau de la synthèse d'urée.

5. Procédé selon la revendication 4, **caractérisé en ce qu'**au moins une partie du dioxyde de carbone comprimé est introduite dans le lavage (5) avec de l'ammoniac liquide et au moins une partie du dioxyde de carbone comprimé est soutirée du complexe d'installations ou la quantité totale du dioxyde de carbone comprimé est soutirée du complexe d'installations ou la quantité totale du dioxyde de carbone comprimé est introduite dans le lavage (5).

6. Procédé selon l'une quelconque des revendications 4 ou 5, **caractérisé en ce qu'**au moins une partie du dioxyde de carbone comprimé est guidée à travers une turbine (14) et l'ammoniac est séparé dans un dispositif pour la séparation d'ammoniac (15) et le dioxyde de carbone purifié (17) est libéré dans l'environnement.

7. Installation pour la préparation d'ammoniac et d'urée, comprenant les éléments suivants se trouvant en liaison fonctionnelle les uns avec les autres :
un dispositif (1) confectionné pour la mise à disposition d'un flux gazeux brut de synthèse comprenant de l'hydrogène, de l'azote et du dioxyde de carbone ainsi que le cas échéant de faibles proportions de méthane, d'argon, de monoxyde de carbone et éventuellement d'hélium ;
au moins un compresseur (4) confectionné pour la compression du flux gazeux brut de synthèse avec formation d'un flux gazeux de synthèse comprimé ;
au moins une colonne de lavage (5) confectionnée pour le lavage du flux gazeux de synthèse comprimé à l'aide d'une composition comprenant de l'ammoniac avec formation d'un flux gazeux de synthèse purifié, appauvri en dioxyde de carbone et d'un condensat ;
au moins un premier réacteur (11) confectionné pour la synthèse d'ammoniac à partir du flux gazeux de synthèse purifié ;
au moins un deuxième réacteur (6) confectionné pour la synthèse d'urée à partir du condensat avec formation d'une composition aqueuse d'urée,
**caractérisé en ce que** l'installation présente en outre au moins un dispositif disposé en aval du compresseur (4) dans le sens d'écoulement, confectionné pour la répartition du flux gazeux de synthèse comprimé provenant du compresseur (4) en un premier flux gazeux de synthèse et en un deuxième flux gazeux de synthèse, une première conduite allant du dispositif de répartition à la colonne de lavage (5), via laquelle première conduite, seul le premier flux gazeux de synthèse peut être introduit dans la colonne de lavage (5), l'installation comprenant en outre un rectificateur (7) et une deuxième conduite allant du dispositif de répartition au rectificateur (7), via laquelle deuxième conduite, seul le deuxième flux gazeux de synthèse peut être introduit dans le rectificateur (7), une conduite partant du deuxième réacteur (6) au rectificateur (7) étant prévue, via laquelle conduite, la composition aqueuse d'urée provenant du deuxième réacteur (6) peut être introduite dans le rectificateur.

8. Installation selon la revendication 7, **caractérisée en ce que** celle-ci comprend en outre un deuxième dispositif de répartition disposé en amont du compresseur (4) dans le sens d'écoulement, au moyen duquel dispositif de répartition le flux gazeux brut de synthèse peut être réparti en deux flux partiels, une première conduite pour un premier flux partiel allant du deuxième dispositif de répartition au compresseur et une deuxième conduite pour un troisième flux partiel allant du deuxième dispositif de répartition à un dispositif de lavage (9), qui est confectionné pour le lavage du troisième flux partiel du gaz brut de synthèse à l'aide d'une solution de lavage comprenant de l'eau et de l'ammoniac avec formation d'un flux gazeux de synthèse prépurifié appauvri en dioxyde de carbone et d'une solution de lavage enrichie en dioxyde de carbone et pour éliminer le dioxyde de carbone de la solution de lavage enrichie en dioxyde de carbone avec formation d'un flux comprimé de dioxyde de carbone, des moyens étant en outre prévus pour rassembler le troisième flux gazeux partiel de synthèse prépurifié provenant du dispositif de lavage (9) en aval du dispositif de lavage (9) et en amont du compresseur avec le premier flux gazeux partiel de synthèse avec formation d'un flux gazeux de synthèse rassemblé, qui peut être introduit via une conduite dans le compresseur (4).

9. Installation selon la revendication 7 ou 8, **caractérisée en ce qu'**elle contient en outre les éléments suivants :
au moins une conduite pour l'introduction d'un flux gazeux de rectification provenant du rectificateur (7) dans la colonne de lavage (5) ;
et/ou un dispositif (10) disposé en amont dans la voie d'écoulement du premier réacteur (11) pour la synthèse d'ammoniac, lequel dispositif est confectionné pour la méthanisation (10) d'un flux gazeux de synthèse purifié, appauvri en dioxyde de carbone provenant de la colonne de lavage (5) avec formation d'un flux gazeux pur de synthèse.

10. Installation selon l'une quelconque des revendications 8 ou 9, **caractérisée en ce que** celle-ci comprend en outre une série de dispositifs (14) à (16), confectionnée pour le soutirage et pour la séparation et la récupération d'ammoniac et d'eau à partir d'au moins une partie du flux de dioxyde de carbone comprimé obtenu en aval du dispositif de lavage (9).

11. Utilisation d'une installation selon l'une quelconque des revendications 7 à 10 dans un procédé selon l'une quelconque des revendications 1 à 6.
